# EUROPEAN PATENT SPECIFICATION

(11) **EP 4 441 079 B1**
(45) Date of publication and mention of the grant of the patent: **11.02.2026**
(21) Application number: 22822625.4
(22) Date of filing: 29.11.2022
(51) Int. Cl.: C07K 14/315, C07K 1/22

(54) **SWITCHABLE POLYPEPTIDE AND ITS USE FOR GENTLE AFFINITY PURIFICATION**
SCHALTBARES POLYPEPTID UND SEINE VERWENDUNG ZUR SANFTEN AFFINITÄTSREINIGUNG
POLYPEPTIDE COMMUTABLE ET SON UTILISATION POUR LA PURIFICATION EN DOUCEUR PAR AFFINITE

(30) Priority: 30.11.2021 GB 202117283
(43) Date of publication of application: 09.10.2024
(73) Proprietor: Oxford University Innovation Limited, Oxford, Oxfordshire OX2 0JB (GB)
(72) Inventor: HOWARTH, Mark, Oxford Oxfordshire OX2 0JB (GB); ANUAR, Irsyad Khairil, Oxford Oxfordshire OX2 0JB (GB); RAHIKAINEN, Rolle, Oxford Oxfordshire OX2 0JB (GB)
(74) Representative: Dehns
(86) International application number: PCT/GB2022/053017
(87) International publication number: WO 2023/099876

(56) References cited:
- WO-A1-2020/115252
- IRSYAD N. A. KHAIRIL ANUAR ET AL: "Spy&Go purification of SpyTag-proteins using pseudo-SpyCatcher to access an oligomerization toolbox", NATURE COMMUNICATIONS, vol. 10, no. 1, 15 April 2019 (2019-04-15), pages 1734, XP055657572, DOI: 10.1038/s41467-019-09678-w
- MASAYUKI TSUKAMOTO ET AL: "Engineered protein A ligands, derived from a histidine-scanning library, facilitate the affinity purification of IgG under mild acidic conditions", JOURNAL OF BIOLOGICAL ENGINEERING, BIOMED CENTRAL LTD, LO, vol. 8, no. 1, 1 July 2014 (2014-07-01), pages 15, XP021195003, ISSN: 1754-1611, DOI: 10.1186/1754-1611-8-15

## Description

### FIELD OF THE INVENTION

The present invention relates to an affinity purification system comprising a polypeptide (protein) that binds selectively (e.g. specifically) and reversibly to its cognate peptide tag (ligand). In particular, the affinity purification system of the invention may be viewed as a two-part system comprising a polypeptide and its cognate peptide tag (affinity tag) that are capable of forming a stable and reversible non-covalent complex (i.e. a polypeptide:ligand complex) that can be dissociated under appropriate conditions (e.g. acidic pH and/or above room temperature, such as 25-45 °C) to facilitate the purification of a molecule or component (e.g. fusion partner) conjugated or fused to said peptide tag. Nucleic acid molecules encoding said polypeptide, vectors comprising said nucleic acid molecules, and host cells comprising said vectors and nucleic acid molecules are also provided. An apparatus comprising said polypeptide immobilised on a solid substrate and a kit for preparing a solid substrate (e.g. resin and/or beads) on which the polypeptide is immobilised are also provided. A process for purifying or isolating a molecule or component using the affinity purification system is also provided.

### BACKGROUND TO THE INVENTION

Affinity chromatography is a central enabling technology for research and for production of therapeutics, vaccines and diagnostics. However, a persistent problem with affinity tags is paradoxically the tags themselves, since the tags often perform no purpose post-purification. Tags, particularly peptide tags, can inhibit crystallisation, interfere with protein interactions, and produce an unhelpful immune response *in vivo.* Tags may be removed by proteolysis but this extra step is timeconsuming, often inefficient and reduces overall yield of the desired product.

There is already a multitude of affinity tags. However, each tag presents its own limitations. The most widely-used, the His-tag, is small and allows costeffective purification. However, there are many examples of His-tagging disrupting protein solubility, structure and function, with particular challenges for proteins that require metal ions for their function in downstream biochemical assays and with the substantial immunogenicity of the His-tag. The four-amino acid C-tag is less immunogenic but is only functional at the C-terminus. Apart from purification, it would be desirable to use peptide tags for assembly or immobilisation, but the low stability of peptide interactions is frequently limiting.

Proteins that are capable of spontaneous isopeptide bond formation have been used to develop peptide tag/binding partner pairs which covalently bind to each other and provide irreversible interactions (see e.g. WO2011/098772, WO 2016/193746, WO2018/197854 and WO2020/183198). In this respect, proteins which are capable of spontaneous isopeptide bond formation may be expressed as separate fragments, to give a peptide tag and a polypeptide binding partner for the peptide tag, where the two fragments are capable of covalently reconstituting by isopeptide bond formation, thereby linking molecules or components fused to the peptide tag and its polypeptide binding partner. The isopeptide bond formed by the peptide tag and its polypeptide binding partner is stable under conditions where non-covalent interactions would rapidly dissociate, e.g. over long periods of time (e.g. weeks), at high temperature (to at least 95 °C), at high force, or with harsh chemical treatment (e.g. pH 2-11, organic solvent, detergents or denaturants).

In brief, a peptide tag and its polypeptide binding partner (a so-called peptide tag/binding partner pair) may be derived from a protein capable of spontaneously forming an isopeptide bond (an isopeptide protein), wherein the domains of the protein are expressed separately to produce a peptide tag that comprises one of the residues involved in the isopeptide bond (e.g. an aspartate) and a polypeptide binding partner (or "catcher") that comprises the other residue involved in the isopeptide bond (e.g. a lysine) and at least one other residue required to form the isopeptide bond (e.g. a glutamate). Mixing the peptide tag and binding partner results in the spontaneous formation of an isopeptide bond between the tag and binding partner. Thus, by separately fusing the peptide tag and binding partner to different molecules or components, e.g. proteins, it is possible to covalently link said molecules or components together via an isopeptide bond formed between the peptide tag and binding partner, i.e. to form a linker between the molecules or components fused to the peptide tag and binding partner.

A peptide tag/binding partner pair, termed SpyTag/SpyCatcher, has been derived from the CnaB2 domain of the *Streptococcus pyogenes* FbaB protein (Zakeri et al., 2012, Proc Natl Acad Sci U S A 109, E690-697) and used in diverse applications, including biomaterials (Botyanszki et al., 2015, Biotechnology and bioengineering 112, 2016-2024; Chen et al., 2014, Proc Natl Acad Sci U S A 108, 11399-11404), next generation sequencing (Stranges et al., 2016, Proc Natl Acad Sci U S A 113, E6749-E6756), enzyme stabilisation (Schoene et al., 2016, Scientific Reports 6, 21151) and vaccine development (Brune et al., 2016, Scientific Reports 6, 19234; Thrane et al., 2016, Journal of Nanobiotechnology 14, 30). Peptide tag/binding partner pairs with improved reaction rates, termed SpyTag002/SpyCatcher002 and SpyTag003/SpyCatcher003, have also been described (WO2018/197854 and WO2020/183198).

Until recently these SpyTag/SpyCatcher systems still required the use of a separate purification tag (e.g. His-tag or C-tag) to enable isolation of each component for reaction or for isolation of the subsequent reaction product, i.e. the SpyTag/SpyCatcher isopeptide bond conjugate. However, using additional purification tags in this manner increases the cloning effort and adds immunogenic sequences which may need to be removed before use in some applications, e.g. for vaccine generation. Thus, the inventors developed a system that can provide high affinity reversible binding of SpyTag peptides to allow for purification of molecules comprising SpyTag peptides, termed SpyDock (WO2020/115252). However, it was found that SpyTag binds so tightly to SpyDock that stringent conditions were needed for elution (e.g. 2.5 M imidazole or pH 2). These stringent conditions limit the utility of SpyDock, as they are likely to lead to global destabilisation of the protein, which is likely to perturb many proteins to which the SpyTag is fused. Moreover, new versions of SpyTag have been developed that bind more tightly to their corresponding Catcher polypeptides, which may impact on the efficacy of their purification using SpyDock. Accordingly, there is a need for new systems and methods for purifying molecules fused to SpyTag peptides that are applicable to the full range of SpyTag peptides and a greater range of targets, such as proteins whose function is disrupted by harsh conditions, e.g. the elution conditions associated with SpyDock.

### SUMMARY OF THE INVENTION

The present inventors have now developed an improved affinity purification system (e.g. an affinity chromatography process) for SpyTag peptides. In particular, the system facilitates the efficient purification of molecules fused to SpyTag peptides under gentler elution conditions than those required for SpyDock and that are suitable for the purification of sensitive proteins, such as viral glycoprotein vaccine targets. Moreover, the improved system is applicable to a range of SpyTag peptides, including SpyTag002 and SpyTag003. Thus, the new system further enhances the dual functionality of SpyTag peptides, i.e. to serve as a purification tag in addition to the current conjugation tag function. This avoids the need to modify SpyTag fusion proteins with additional affinity tags, which may need to be removed from the SpyTag/SpyCatcher reaction product post-purification, e.g. to avoid immunogenicity against the affinity tag.

It has surprisingly been determined that selective and specific modification of the SpyDock polypeptide to replace various amino acid residues with histidine residues results in a polypeptide that provides highly specific non-covalent binding of all SpyTag peptides that can be reversed by altering the pH conditions to a weakly acidic pH (i.e. about 5.0), i.e. a pH-switchable SpyTag binding protein. Unexpectedly, the inventors further determined that the specific binding of the modified SpyDock polypeptide to SpyTag peptides was temperature-sensitive within a physiological temperature range (e.g. enabling elution at about 37 °C). Moreover, following the design and implementation of an innovative phage selection strategy, the activity of the modified SpyDock polypeptide was further improved by the selection and introduction of additional amino acid substitutions, resulting in a polypeptide termed "SpySwitch" (SEQ ID NO: 6).

As shown in detail in the Examples, the inventors have shown that the modified SpyDock polypeptides (e.g. SpyDock2.0 (SEQ ID NO: 10) and SpySwitch (SEQ ID NO: 6)) enable purification of polypeptides conjugated to SpyTag peptides (SpyTag (SEQ ID NO: 3) and SpyTag003 (SEQ ID NO: 5)) under mild conditions suitable for a wide range of target proteins, e.g. capture at neutral pH followed by elution under mildly acidic conditions (e.g. about pH 4.0-6.0) or physiological temperatures (e.g. about 30-40 °C, preferably about 35-40 °C). Moreover, purification with SpySwitch was shown to give higher purity than purification with either SpyDock or His-tag:nickel-nitrilotriacetic acid (Ni-NTA) purification.

Advantageously, the inventors also determined that the polypeptide of the invention immobilised on a solid substrate is stable in long-term storage under suitable conditions, e.g. aseptic (e.g. anti-bacterial) conditions such as in 20% ethanol at 10 °C or less (e.g. about 4 °C). Moreover, the inventors have shown that a solid substrate on which the polypeptide of the invention is immobilised may be regenerated and re-used without significant loss of purification activity.

Thus, in one aspect, the present invention provides a polypeptide comprising:
(i) an amino acid sequence as set forth in SEQ ID NO: 1, wherein X at position 77 is selected from alanine, glycine, serine, asparagine or threonine;
(ii) a portion of (i) comprising an amino acid sequence as set forth in SEQ ID NO: 2, wherein X at position 56 is selected from alanine, glycine, serine, asparagine or threonine;
(iii) an amino acid sequence with at least 80% sequence identity to a sequence as set forth in SEQ ID NO: 1, wherein the polypeptide comprises alanine, glycine, serine, asparagine or threonine at a position equivalent to position 77 of SEQ ID NO: 1, and wherein the polypeptide comprises histidine at positions equivalent to positions 30, 84 and 85 of SEQ ID NO: 1; or
(iv) a portion of (iii) comprising an amino acid sequence with at least 80% sequence identity to a sequence as set forth in SEQ ID NO: 2, wherein the polypeptide comprises alanine, glycine, serine, asparagine or threonine at a position equivalent to position 56 of SEQ ID NO: 2, and wherein the polypeptide comprises histidine at positions equivalent to positions 9, 63 and 64 of SEQ ID NO: 2,
wherein the polypeptide binds selectively and reversibly to a peptide comprising an amino acid sequence as set forth in SEQ ID NO: 3, 4 or 5.

In another aspect, the invention provides a nucleic acid molecule comprising a nucleotide sequence which encodes a polypeptide of the invention.

In yet another aspect, the invention provides a vector comprising the nucleic acid molecule of the invention.

In a further aspect, the invention provides a cell comprising the nucleic acid molecule or vector of the invention.

As noted above, the polypeptide of the invention finds utility in the isolation and/or purification of molecules or components comprising a SpyTag peptide. Thus, in another aspect, the invention provides a process for purifying or isolating a molecule or component comprising a peptide having an amino acid sequence with at least 80% sequence identity to a sequence as set forth in one of SEQ ID NOs: 3-5, preferably wherein said peptide comprises an aspartic acid at a position equivalent to position 7 of SEQ ID NO: 3, position 8 of SEQ ID NO: 4 or position 10 of SEQ ID NO: 5, said process comprising:
a) providing a solid substrate on which a polypeptide of the invention is immobilised;
b) providing a sample comprising said molecule or component;
c) contacting the solid substrate of a) with the sample of b) under conditions that enable said peptide to selectively bind to said polypeptide, thereby forming a non-covalent complex between said polypeptide immobilised on the solid substrate and molecule or component comprising said peptide;
d) washing the solid substrate with a wash solution; and
e) separating the molecule or component comprising the peptide from the polypeptide immobilised on the solid substrate.

Alternatively viewed, the invention relates to the use of a polypeptide of the invention to purify or isolate a molecule or component comprising a peptide having an amino acid sequence with at least 80% sequence identity to a sequence as set forth in one of SEQ ID NOs: 3-5, preferably wherein said peptide comprises an aspartic acid at a position equivalent to position 7 of SEQ ID NO: 3, position 8 of SEQ ID NO: 4 or position 10 of SEQ ID NO: 5.

In yet another aspect, the invention provides an apparatus suitable for use in the process or use of the invention comprising a solid substrate on which a polypeptide of the invention is immobilised.

The invention also provides a kit suitable for use in preparing a solid substrate on which a polypeptide of the invention is immobilised, comprising:
a) a polypeptide of the invention; and
b) means for immobilising the polypeptide of a) on a solid substrate.

In a further aspect, the invention provides a resin and/or beads for use in protein purification methods on which a polypeptide of the invention is immobilised.

### DETAILED DESCRIPTION

The inventors have previously determined that selected mutations in the SpyCatcher polypeptides at the position of the activating glutamic acid residue in the CnaB2 triad were sufficient to abrogate the formation of an isopeptide bond between SpyCatcher and SpyTag, whilst maintaining a selective, stable and reversible non-covalent interaction (see e.g. WO 2020/115252). Thus, in some embodiments, X at a position equivalent to position 77 of SEQ ID NO: 1 or position 56 of SEQ ID NO: 2 is selected from alanine, glycine or serine and preferably is alanine.

In a preferred embodiment, the polypeptide comprises alanine at a position equivalent to position 77 of SEQ ID NO: 1, such that the polypeptide comprises an amino acid sequence as set forth in SEQ ID NO: 6.

As mentioned above, the inventors have determined that the mutation of particular residues of the SpyDock polypeptide (SEQ ID NO: 15) improved its utility in affinity purification via the non-covalent interaction between the polypeptide of the invention and SpyTag peptides (e.g. SEQ ID NOs: 3, 4 or 5).

Thus, in some embodiments, the polypeptide of the invention defined above comprises histidine at one or both positions equivalent to positions 28 and 32 of SEQ ID NO: 1 (i.e. at one or both positions equivalent to positions 7 and 11 of SEQ ID NO: 2).

In some embodiments the polypeptide of the invention defined above comprises one or both of: (i) a glutamic acid at a position equivalent to position 91 of SEQ ID NO: 1 (i.e. at a position equivalent to position 70 of SEQ ID NO: 2); and (ii) an aspartic acid at a position equivalent to position 103 of SEQ ID NO: 1 (i.e. at a position equivalent to position 82 of SEQ ID NO: 2).

In some embodiments the polypeptide of the invention defined above comprises one or more of: (i) a threonine at a position equivalent to position 59 of SEQ ID NO: 1 (i.e. at a position equivalent to position 38 of SEQ ID NO: 2); (ii) an isoleucine at a position equivalent to position 94 of SEQ ID NO: 1 (i.e. at a position equivalent to position 73 of SEQ ID NO: 2); and (iii) a proline at a position equivalent to position 111 of SEQ ID NO: 1 (i.e. at a position equivalent to position 90 of SEQ ID NO: 2).

Thus, in a particular embodiment, the polypeptide comprises an amino acid sequence with at least 80% sequence identity to a sequence as set forth in SEQ ID NO: 1 and wherein the amino acid sequence comprises alanine, glycine, serine, asparagine or threonine at a position equivalent to position 77 of SEQ ID NO: 1, histidine at positions equivalent to positions 30, 84 and 85 of SEQ ID NO: 1, a glutamic acid at a position equivalent to position 91 of SEQ ID NO: 1, and an aspartic acid at a position equivalent to position 103 of SEQ ID NO: 1 and optionally one or more (e.g. 2, 3, 4 or 5) of the following:
i) histidine at a position equivalent to position 28 of SEQ ID NO: 1;
ii) histidine at a position equivalent to position 32 of SEQ ID NO: 1;
iii) threonine at a position equivalent to position 59 of SEQ ID NO: 1;
iv) isoleucine at a position equivalent to position 94 of SEQ ID NO: 1; and
v) proline at a position equivalent to position 111 of SEQ ID NO: 1,
wherein the polypeptide binds selectively and reversibly to a peptide comprising an amino acid sequence as set forth in SEQ ID NO: 3, 4 or 5.

Thus, in another embodiment, the polypeptide comprises an amino acid sequence with at least 80% sequence identity to a sequence as set forth in SEQ ID NO: 2 and wherein the amino acid sequence comprises alanine, glycine, serine, asparagine or threonine at a position equivalent to position 56 of SEQ ID NO: 2, histidine at positions equivalent to positions 9, 63 and 64 of SEQ ID NO: 2, a glutamic acid at a position equivalent to position 70 of SEQ ID NO: 2, and an aspartic acid at a position equivalent to position 82 of SEQ ID NO: 2 and optionally one or more (e.g. 2, 3, 4 or 5) of the following:
i) histidine at a position equivalent to position 7 of SEQ ID NO: 2;
ii) histidine at a position equivalent to position 11 of SEQ ID NO: 2;
iii) threonine at a position equivalent to position 38 of SEQ ID NO: 2;
iv) isoleucine at a position equivalent to position 73 of SEQ ID NO: 2; and
v) proline at a position equivalent to position 90 of SEQ ID NO: 2,
wherein the polypeptide binds selectively and reversibly to a peptide comprising an amino acid sequence as set forth in SEQ ID NO: 3, 4 or 5.

Thus, in another particular embodiment, the polypeptide comprises an amino acid sequence with at least 80% sequence identity to a sequence as set forth in SEQ ID NO: 1 and wherein the amino acid sequence comprises alanine, glycine, serine, asparagine or threonine at a position equivalent to position 77 of SEQ ID NO: 1, histidine at positions equivalent to positions 28, 30, 32, 84 and 85 of SEQ ID NO: 1, a glutamic acid at a position equivalent to position 91 of SEQ ID NO: 1, and an aspartic acid at a position equivalent to position 103 of SEQ ID NO: 1 and one or more (e.g. 2 or 3) of the following:
i) threonine at a position equivalent to position 59 of SEQ ID NO: 1;
ii) isoleucine at a position equivalent to position 94 of SEQ ID NO: 1; and
iii) proline at a position equivalent to position 111 of SEQ ID NO: 1,
wherein the polypeptide binds selectively and reversibly to a peptide comprising an amino acid sequence as set forth in SEQ ID NO: 3, 4 or 5.

Thus, in another particular embodiment, the polypeptide comprises an amino acid sequence with at least 80% sequence identity to a sequence as set forth in SEQ ID NO: 2 and wherein the amino acid sequence comprises alanine, glycine, serine, asparagine or threonine at a position equivalent to position 56 of SEQ ID NO: 2, histidine at positions equivalent to positions 7, 9, 11, 63 and 64 of SEQ ID NO: 2, a glutamic acid at a position equivalent to position 70 of SEQ ID NO: 2, and an aspartic acid at a position equivalent to position 82 of SEQ ID NO: 2 and one or more (e.g. 2 or 3) of the following:
i) threonine at a position equivalent to position 38 of SEQ ID NO: 2;
ii) isoleucine at a position equivalent to position 73 of SEQ ID NO: 2; and
iii) proline at a position equivalent to position 90 of SEQ ID NO: 2,
wherein the polypeptide binds selectively and reversibly to a peptide comprising an amino acid sequence as set forth in SEQ ID NO: 3, 4 or 5.

Thus, in a particular preferred embodiment, the polypeptide comprises:
(A) an amino acid sequence with at least 80% sequence identity to a sequence as set forth in SEQ ID NO: 1 and wherein the amino acid sequence comprises alanine, glycine, serine, asparagine or threonine at a position equivalent to position 77 of SEQ ID NO: 1 and all of the following:
   i) histidine at positions equivalent to positions 28, 30, 32, 84 and 85 of SEQ ID NO: 1;
   ii) threonine at a position equivalent to position 59 of SEQ ID NO: 1;
   iii) glutamic acid at a position equivalent to position 91 of SEQ ID NO: 1;
   iv) isoleucine at a position equivalent to position 94 of SEQ ID NO: 1;
   v) aspartic acid at a position equivalent to position 103 of SEQ ID NO: 1; and
   vi) proline at a position equivalent to position 111 of SEQ ID NO: 1, or
(B) an amino acid sequence with at least 80% sequence identity to a sequence as set forth in SEQ ID NO: 2 and wherein the amino acid sequence comprises alanine, glycine, serine, asparagine or threonine at a position equivalent to position 56 of SEQ ID NO: 2 and all of the following:
   i) histidine at positions equivalent to positions 7, 9, 11, 63 and 64 of SEQ ID NO: 2;
   ii) threonine at a position equivalent to position 38 of SEQ ID NO: 2;
   iii) glutamic acid at a position equivalent to position 70 of SEQ ID NO: 2;
   iv) isoleucine at a position equivalent to position 73 of SEQ ID NO: 2;
   v) aspartic acid at a position equivalent to position 82 of SEQ ID NO: 2; and
   vi) proline at a position equivalent to position 90 of SEQ ID NO: 2,
wherein the polypeptide binds selectively and reversibly to a peptide comprising an amino acid sequence as set forth in SEQ ID NO: 3, 4 or 5.

The inventors have previously determined that selected residues in the SpyDock polypeptide may contribute to the efficacy of the reversible non-covalent interaction with SpyTag peptides and it may be advantageous to retain the residues in the polypeptide of the invention. Thus, in some embodiments, the polypeptide of the invention defined above comprises a glutamic acid at a position equivalent to position 108 of SEQ ID NO: 1 (i.e. at a position equivalent to position 87 of SEQ ID NO: 2).

In some embodiments, the polypeptide of the invention defined above comprises a proline at a position equivalent to position 89 of SEQ ID NO: 1 (i.e. at a position equivalent to position 68 of SEQ ID NO: 2).

In some embodiments, the polypeptide of the invention defined above comprises an aspartic acid at a position equivalent to position 97 of SEQ ID NO: 1 (i.e. at a position equivalent to position 76 of SEQ ID NO: 2).

In some embodiments, the polypeptide of the invention defined above comprises a lysine at a position equivalent to position 31 of SEQ ID NO: 1 (i.e. at a position equivalent to position 10 of SEQ ID NO: 2).

Thus, in a particular embodiment, the polypeptide comprises:
(i) an amino acid sequence with at least 80% sequence identity to a sequence as set forth in SEQ ID NO: 1, wherein the polypeptide comprises alanine, glycine, serine, asparagine or threonine at a position equivalent to position 77 of SEQ ID NO: 1, histidine at positions equivalent to positions 30, 84 and 85 of SEQ ID NO: 1 and one or more (preferably all) of the following:
   (1) lysine at a position equivalent to position 31 of SEQ ID NO: 1;
   (2) proline at a position equivalent to position 89 of SEQ ID NO: 1;
   (3) aspartic acid at a position equivalent to position 97 of SEQ ID NO: 1; and
   (4) glutamic acid at a position equivalent to position 108 of SEQ ID NO: 1; or
(ii) a portion of (i) comprising an amino acid sequence with at least 80% sequence identity to a sequence as set forth in SEQ ID NO: 2, wherein the polypeptide comprises alanine, glycine, serine, asparagine or threonine at a position equivalent to position 56 of SEQ ID NO: 2, histidine at positions equivalent to positions 9, 63 and 64 of SEQ ID NO: 2 and one or more (preferably all) of the following:
   (1) lysine at a position equivalent to position 10 of SEQ ID NO: 2;
   (2) proline at a position equivalent to position 68 of SEQ ID NO: 2;
   (3) aspartic acid at a position equivalent to position 76 of SEQ ID NO: 2; and
   (4) glutamic acid at a position equivalent to position 87 of SEQ ID NO: 2,
wherein the polypeptide binds selectively and reversibly to a peptide comprising an amino acid sequence as set forth in SEQ ID NO: 3, 4 or 5.

In some embodiments the polypeptide comprises an amino acid sequence with at least 80% sequence identity to a sequence as set forth in SEQ ID NO: 1, wherein the amino acid sequence comprises alanine, glycine, serine, asparagine or threonine at position 77, histidine at positions 30, 84 and 85 of SEQ ID NO: 1, lysine at position 31, proline at position 89, aspartic acid at position 97, glutamic acid at position 108 and one or more of the following:
i) threonine at position 2;
ii) glycine at position 9;
iii) proline at position 13;
iv) threonine at position 19;
v) arginine at position 37;
vi) histidine at position 62;
vii) glutamic acid at position 105; and
viii) threonine at position 113,
wherein the specified amino acid residues are at positions equivalent to the positions in SEQ ID NO: 1.

In some embodiments, the polypeptide comprises an amino acid sequence with at least 80% sequence identity to a sequence as set forth in SEQ ID NO: 2 and wherein the amino acid sequence comprises alanine, glycine, serine, asparagine or threonine at position 56, histidine at positions 9, 63 and 64, lysine at position 10, proline at position 68, aspartic acid at position 76, glutamic acid at position 87 and one or more of the following:
i) arginine at position 16;
ii) histidine at position 41;
iii) glutamic acid at position 84; and
iv) threonine at position 92,
wherein the specified amino acid residues are at positions equivalent to the positions in SEQ ID NO: 2.

Thus, in some embodiments the polypeptide comprises an amino acid sequence with at least 80% sequence identity to a sequence as set forth in SEQ ID NO: 1, wherein the amino acid sequence comprises alanine, glycine, serine, asparagine or threonine at position 77, histidine at positions 30, 84 and 85, lysine at position 31, proline at position 89, aspartic acid at position 97, glutamic acid at position 108 and any two, three, four, five, six, seven or eight of the following:
i) threonine at position 2;
ii) glycine at position 9;
iii) proline at position 13;
iv) threonine at position 19;
v) arginine at position 37;
vi) histidine at position 62;
vii) glutamic acid at position 105; and
viii) threonine at position 113,
wherein the specified amino acid residues are at positions equivalent to the positions in SEQ ID NO: 1.

In some embodiments, the polypeptide comprises an amino acid sequence with at least 80% sequence identity to a sequence as set forth in SEQ ID NO: 2 and wherein the amino acid sequence comprises alanine, glycine, serine, asparagine or threonine at position 56, histidine at positions 9, 63 and 64, lysine at position 10, proline at position 68, aspartic acid at position 76, glutamic acid at position 87 and any two, three or four of the following:
i) arginine at position 16;
ii) histidine at position 41;
iii) glutamic acid at position 84; and
iv) threonine at position 92,
wherein the specified amino acid residues are at positions equivalent to the positions in SEQ ID NO: 2.

It is contemplated that the polypeptide of the invention may comprise any one or any combination of the specified amino acid residues defined above as long as the polypeptide satisfies the functional requirements of the invention, i.e. the polypeptide binds selectively and reversibly to a peptide comprising an amino acid sequence as set forth in SEQ ID NO: 3, 4 or 5.

However, in particularly preferred embodiments the polypeptide comprises:
(A) an amino acid sequence with at least 80% sequence identity to a sequence as set forth in SEQ ID NO: 1, wherein the amino acid sequence comprises alanine, glycine, serine, asparagine or threonine (preferably alanine) at a position equivalent to position 77 of SEQ ID NO: 1 and all of the following:
   i) histidine at positions equivalent to positions 28, 30, 32, 62, 84 and 85 of SEQ ID NO: 1;
   ii) threonine at positions equivalent to positions 2, 19, 59 and 113 of SEQ ID NO: 1;
   iii) glycine at a position equivalent to position 9 of SEQ ID NO: 1;
   iv) glutamic acid at positions equivalent to positions 91, 105 and 108 of SEQ ID NO: 1;
   v) isoleucine at a position equivalent to position 94 of SEQ ID NO: 1;
   vi) aspartic acid at positions equivalent to positions 97 and 103 of SEQ ID NO: 1;
   vii) proline at positions equivalent to positions 13, 89 and 111 of SEQ ID NO: 1;
   viii) lysine at a position equivalent to position 31 of SEQ ID NO: 1; and
   ix) arginine at a position equivalent to position 37 of SEQ ID NO: 1; or
(B) an amino acid sequence with at least 80% sequence identity to a sequence as set forth in SEQ ID NO: 2 and wherein the amino acid sequence comprises alanine, glycine, serine, asparagine or threonine (preferably alanine) at a position equivalent to position 56 of SEQ ID NO: 2 and all of the following:
   i) histidine at positions equivalent to positions 7, 9, 11, 41, 63 and 64 of SEQ ID NO: 2;
   ii) threonine at positions equivalent to positions 38 and 92 of SEQ ID NO: 2;
   iii) glutamic acid at positions equivalent to positions 70, 84 and 87 of SEQ ID NO: 2;
   iv) isoleucine at a position equivalent to position 73 of SEQ ID NO: 2;
   v) aspartic acid at positions equivalent to positions 76 and 82 of SEQ ID NO: 2;
   vi) proline at positions equivalent to positions 68 and 90 of SEQ ID NO: 2;
   vii) lysine at a position equivalent to position 10 of SEQ ID NO: 2; and
   ix) arginine at a position equivalent to position 16 of SEQ ID NO: 2.

The inventors have determined that the presence of a cysteine residue in the polypeptide of the invention advantageously enables efficient coupling of the polypeptide to a solid substrate with minimal disruption to SpyTag peptide (SEQ ID NO: 3, 4 or 5) non-covalent binding. Thus, in some embodiments, the polypeptide variants defined above may also comprise a cysteine at a position equivalent to position 49 in SEQ ID NO: 1 or position 28 in SEQ ID NO: 2.

The polypeptide of the invention binds selectively and reversibly to its cognate peptide tag (i.e. SpyTag peptide or a variant thereof), such as a peptide comprising an amino acid sequence as set forth in SEQ ID NOs: 3, 4 or 5, under suitable conditions.

The term "binds selectively" refers to the ability of the polypeptide to bind non-covalently (e.g. by van der Waals forces and/or hydrogen-bonding) to its cognate peptide tag with greater affinity and/or specificity than to other components in the sample in which the peptide tag is present (e.g. the sample from which the peptide tag (and associated molecule or component to which the peptide tag is fused or conjugated, i.e. fusion partner) is to be isolated or purified). Thus, the polypeptide of the invention may alternatively be viewed as binding specifically and reversibly to its cognate peptide tag (i.e. SpyTag peptide or a variant thereof), such as a peptide comprising or consisting of an amino acid sequence as set forth in SEQ ID NOs: 3, 4 or 5, under suitable conditions.

Binding to the cognate peptide tag may be distinguished from binding to other molecules (e.g. peptides or polypeptides) present in the sample, i.e. non-cognate molecules. The polypeptide of the invention either does not bind to other molecules (e.g. peptides or polypeptides) present in the sample or does so negligibly or non-detectably so that any such non-specific binding, if it occurs, readily may be distinguished from binding to the cognate peptide tag.

In particular, if the polypeptide of the invention binds to molecules other than the cognate peptide tag, such binding must be transient and the binding affinity must be less than the binding affinity of the polypeptide for the cognate peptide tag. Thus, the binding affinity of the polypeptide for the peptide tag should be at least an order of magnitude more than the other molecules (i.e. non-cognate molecules) present in the sample. Preferably, the binding affinity of the polypeptide for the cognate peptide tag should be at least 2, 3, 4, 5, or 6 orders of magnitude more than the binding affinity for non-cognate molecules (e.g. peptides or polypeptides).

Thus, selective or specific binding refers to affinity of the polypeptide of the invention for its cognate peptide tag where the dissociation constant of the polypeptide for the cognate peptide tag is less than about 10⁻³ M. In a preferred embodiment the dissociation constant of the polypeptide for its cognate peptide tag is less than about 10⁻⁴ M, 10⁻⁵ M, 10⁻⁶ M, 10⁻⁷ M, 10⁻⁸ M or 10⁻⁹ M.

The binding selectivity (e.g. specificity) of the polypeptide of the invention may also be defined based on the yield and/or purity of the product, i.e. the cognate peptide tag and associated molecule or component (fusion partner, e.g. polypeptide), to which the peptide tag is fused or conjugated, obtained in the isolation or purification process defined herein. In some embodiments, the polypeptide of the invention in the process defined herein results in a product with a purity of at least about 75%, such as at least about 80%, 85%, 90%, 95%, 96%, 97% or 98%. The purity of the product obtained using the process and polypeptide of the invention may be determined using any suitable means, such as the SDS-PAGE method described in the Examples below.

In some embodiments, the polypeptide of the invention in the process defined herein results in a product with a yield of at least about 50%, such as about 60%, 70%, 75%, 80% 85% or 90%. The yield of the product obtained using the process and polypeptide of the invention may be determined using any suitable means.

The term "cognate" refers to components that function or specifically interact together. Thus, in the context of the present invention, a cognate pair refers to a peptide tag (i.e. SpyTag or a variant thereof, such as a peptide comprising or consisting of SEQ ID NOs: 3, 4 and 5) and the polypeptide of the invention that bind non-covalently to form a complex (i.e. a polypeptide:peptide tag complex).

Thus, a cognate peptide tag refers to a SpyTag peptide or variant thereof (e.g. a peptide comprising an amino acid sequence set forth in one of SEQ ID NOs: 3-5) to which the polypeptide of the invention can bind selectively (e.g. specifically) and reversibly. In some embodiments, the cognate peptide tag may be a peptide comprising an amino acid sequence with at least 80% sequence identity to an amino acid sequence as set forth in one of SEQ ID NOs: 3-5.

In a preferred embodiment, the cognate peptide tag is capable of spontaneously forming an isopeptide bond with a "SpyCatcher" polypeptide, e.g. a polypeptide comprising an amino acid sequence as set forth in SEQ ID NO: 11, 12 or 13, e.g. between an aspartic acid in the cognate peptide tag (i.e. an aspartic acid at position equivalent to position 7 in SEQ ID NO: 3, position 8 in SEQ ID NO: 4 or position 10 in SEQ ID NO: 5) and the lysine residue at position 31 of SEQ ID NO: 11, 12 or 13. In some embodiments, the cognate peptide tag may not be capable of spontaneously forming an isopeptide bond with a polypeptide comprising an amino acid sequence as set forth in SEQ ID NO: 11, 12 or 13, e.g. because it does not contain an aspartic acid residue capable of reacting with the lysine residue at position 31 of SEQ ID NO: 11, 12 or 13. Such cognate peptide tags may find utility as negative control peptides in the isolation or purification process of the invention, described below.

Thus, a polypeptide of the invention must bind selectively and reversibly to at least one peptide comprising or consisting of an amino acid sequence as set forth in SEQ ID NOs: 3-5. In a preferred embodiment, polypeptide of the invention must bind selectively and reversibly to each peptide comprising or consisting of an amino acid as set forth in SEQ ID NOs: 3-5. Thus, the polypeptide of the invention binds to at least one (preferably all) peptide(s) comprising or consisting of an amino acid sequence as set forth in SEQ ID NOs: 3-5 with greater affinity and/or specificity than to other components in the sample (i.e. non-cognate molecules) in which the peptide tag is present. A sample may be any sample (e.g. cell lysate etc. as described below) from which the peptide tag (and associated molecule or component to which the peptide tag is fused or conjugated, i.e. fusion partner) is to be isolated or purified. However, the polypeptide of the invention may also bind to other cognate peptide tags as defined herein.

Alternatively viewed, the polypeptide variants of the invention (variants of SEQ ID NO: 1 or 6 as defined herein) must be capable of competing with a polypeptide comprising an amino acid sequence set forth in SEQ ID NO: 6 or 16 (SpySwitch) for binding with a cognate peptide tag as defined herein, e.g. one or all of SEQ ID NOs: 3-5. Any suitable competition assay known in the art may be used to determine whether polypeptide variants of the invention compete with SpySwitch.

A non-cognate molecule, particularly a non-cognate peptide or polypeptide may be defined as a peptide or polypeptide that does not contain an amino acid sequence consisting of an amino acid sequence with at least 60% sequence identity to a SpyTag peptide, such as SEQ ID NOs: 3, 4 or 5. Preferably, the non-cognate molecule does not contain consecutive sequence of 12-16 amino acids with more than about 60%, 55%, 50%, 45%, 40%, 35%, 30%, 25% or 20% sequence identity to a SpyTag peptide, such as SEQ ID NOs: 3, 4 or 5. Other non-cognate molecules include carbohydrates, sugars, nucleic acids, lipids, ions and small molecules.

Suitable conditions for the selective or specific binding of the polypeptide to its cognate peptide tag are set out below (e.g. conditions used in step (c) of the process of the invention). However, it is evident from the Examples below that the polypeptide of the invention is able to bind selectively and specifically to its cognate peptide tag under a range of conditions.

For instance, the polypeptide may bind selectively (e.g. specifically) to its cognate peptide tag in a variety of buffers including Tris-HCl, phosphate buffered saline (PBS), 4-(2-hydroxyethyl)-1-piperazineethanesulfonic acid (HEPES), HEPES buffered saline (HBS), Tris-phosphate and Tris buffered saline (TBS), both with and without EDTA. Detergents such as Tween 20 and Triton X-100 may also be present, as may salts such as NaCl or KCI.

As noted above, the inventors have determined that pH and temperature affect the binding of the polypeptide of the invention to its cognate peptide tag. Accordingly, binding may occur at a neutral pH, e.g. a pH of about 6.5-8.5, e.g. about 6.8-8.3, 6.9-8.2, such as about 7.0-8.0, over a range of temperatures, e.g. about 0-25 °C, e.g. 1, 2, 3, 4, 5, 10, 12, 15, 18, 20, 22 or 25 °C, preferably about 0-15 or 0-10 °C, e.g. about 4 °C. The skilled person would readily be able to determine other suitable conditions.

Thus, in some embodiments, conditions that are suitable for selective (e.g. specific) binding between the polypeptide of the invention and its cognate peptide tag include any conditions in which contacting the polypeptide of the invention with its cognate peptide tag (e.g. a sample comprising the cognate peptide tag) results in the formation of non-covalent complex between polypeptide and cognate peptide tag. For instance, contacting said polypeptide and cognate peptide tag in buffered conditions, e.g. in a buffered solution or on a solid phase (e.g. column) that has been equilibrated with a buffer, such as Tris, HEPES, PBS or TBS. The step of contacting may be at a neutral pH, such as pH 6.5-8.5, e.g. 7.0-8.0, such as about pH 6.6, 6.7, 6.8, 6.9, 7.0, 7.1, 7.2, 7.3, 7.4, 7.5, 7.6, 7.7, 7.8, 7.9, 8.0, 8.1 or 8.2, preferably about 7.5. Additionally or alternatively, the step of contacting may be at a temperature of about 0-25 °C, e.g. about 1-24, 2-23, 3-22, 4-21, 5-20, 6-19, 7-18, 8-17, 9-16 or 10-15 °C, e.g. about 2, 4, 6, 8, 10, 12, 15, 18, 20, 22 or 25 °C, preferably about 0-10 or 4-10 °C, e.g. about 4 °C. Any suitable combination of pH and temperature conditions selected from those set out above may be used. For instance, step of contacting may be at a pH of about 7.3-7.7 (e.g. about 7.5) and a temperature of about 0-10 °C (e.g. about 4-8 °C).

The term "reversible" or "binds reversibly" refers to ability of the interaction between the polypeptide and its cognate peptide tag to be disrupted, resulting in the separation (dissociation) of the complex under suitable conditions. In other words, the non-covalent interaction formed by the polypeptide:cognate peptide tag complex can be broken under suitable conditions to enable the separation of the constituent parts. Suitable conditions to dissociate the complex may include any conditions that are able to disrupt or break the non-covalent bonds required to form the complex. An example of suitable conditions that may be used in the process of the invention is set out below.

It will be evident that conditions to dissociate the polypeptide:cognate peptide tag complex preferably should not lead to irreversible loss of activity of the SpyTag peptide and/or fusion partner. For instance, conditions that prevent SpyTag from reacting spontaneously with a SpyCatcher polypeptide to form an isopeptide bond should be avoided. Similarly, conditions that alter or inhibit (e.g. denature) the molecule or component fused to the SpyTag peptide (i.e. fusion partner, e.g. polypeptide) are not suitable for dissociating polypeptide:cognate peptide tag complex, as such conditions would limit the utility of SpyTag fusion in downstream applications. Such conditions will depend on the nature of the fusion partner and the skilled person readily could determine which conditions are suitable (or unsuitable) based on methods known in the art. By way of example, boiling the polypeptide:cognate peptide tag complex and/or treatment with 1% SDS would dissociate the polypeptide:cognate peptide tag complex, but may irreversibly alter (e.g. denature) the fusion partner.

The term "spontaneous" as used herein refers to an isopeptide bond, which can form in a protein or between a peptide and protein, e.g. the cognate peptide tag described herein and a polypeptide comprising an amino acid sequence as set forth in SEQ ID NO: 11, 12 or 13 without any other agent (e.g. an enzyme catalyst) being present and/or without chemical modification of the protein or peptide, e.g. without native chemical ligation or chemical coupling using 1-ethyl-3-(3-dimethylaminopropyl) carbodiimide (EDC). Thus, native chemical ligation to modify a peptide or protein having a C-terminal thioester is not carried out.

Thus, a spontaneous isopeptide bond can form between a cognate peptide tag as described herein and a polypeptide (e.g. a polypeptide comprising an amino acid sequence as set forth in SEQ ID NO: 11, 12 or 13) when in isolation and without chemical modification of the cognate peptide tag and/or the polypeptide with which it reacts. A spontaneous isopeptide bond may therefore form of its own accord in the absence of enzymes or other exogenous substances and without chemical modification of the cognate peptide tag described herein and the polypeptide with which it reacts.

A spontaneous isopeptide bond may form almost immediately after contact of the reactive peptide tag and polypeptide, e.g. within 1, 2, 3, 4, 5, 10, 15, 20, 25 or 30 minutes, or within 1, 2, 4, 8, 12, 16, 20 or 24 hours.

The polypeptide of the invention encompasses mutant forms (i.e. referred to herein as homologues, variants or derivatives), which are structurally similar to the exemplified polypeptides set forth in SEQ ID NOs: 6, 7 and 16. The polypeptide variants of the invention are able to bind selectively and reversibly to the cognate peptide tag under suitable conditions as defined above.

In cases where a polypeptide variant comprises mutations, e.g. deletions or insertions, relative to SEQ ID NOs: 6 and 7, the residues specified above are present at equivalent amino acid positions in the variant polypeptide sequences. In some embodiments, deletions in the polypeptide variants of the invention are not N-terminal and/or C-terminal truncations.

However, as mentioned above, it is contemplated that the polypeptide exemplified herein (e.g. SEQ ID NO: 6) and variants thereof may be truncated at the N-terminus and/or C-terminus without eliminating the activity of the polypeptide. In particular, SEQ ID NO: 6 may be truncated by up to 21 amino acids at the N-terminus to provide a polypeptide as set forth in SEQ ID NO: 7. Additionally or alternatively, in some embodiments the polypeptide set forth in SEQ ID NO: 6 may be truncated by less than 21 amino acids, e.g. 5, 10, 15 or 20 amino acids. In some embodiments, the polypeptide and portion exemplified herein (i.e. SEQ ID NOs: 6 and 7, respectively) may be truncated by up to 9 amino acids at the C-terminus (e.g. 1, 2, 3, 4, 5, 6, 7, 8 or 9 amino acids), preferably by 8 amino acids or fewer. Thus, the term variant as used herein includes truncation variants of the exemplified polypeptides. In a particularly preferred embodiment, the truncated variant polypeptide of the invention comprises an amino acid sequence as set forth in SEQ ID NO: 7 or a variant thereof (e.g. a polypeptide with at least 80% sequence identity to an amino acid sequence as set forth in SEQ ID NO: 2 or 7), as discussed above.

As referred to herein a "portion" comprises at least an amino acid sequence as set forth in SEQ ID NO: 7 optionally further truncated at the C-terminal end, i.e. at least 83, 84, 85, 86, 87, 88, 89, 90, 95, 100, 105, 110 or more amino acids of SEQ ID NO: 6 (the sequence from which it is derived), preferably containing an amino acid sequence as set forth in SEQ ID NO: 7. Thus, said portion may be obtained from a central or N-terminal or C-terminal portion of the sequence. Preferably said portion is obtained from the central portion, i.e. it comprises an N-terminal and/or C-terminal truncation as defined above, preferably an N-terminal truncation. Notably, "portions" as described herein are polypeptides of the invention and therefore satisfy the identity (relative to a comparable region) conditions and functional equivalence conditions mentioned herein.

An equivalent position in the polypeptide of the invention is preferably determined by reference to the amino acid sequence of SEQ ID NO: 1 or 6. The homologous or corresponding position can be readily deduced by lining up the sequence of the homologue (mutant, variant or derivative) polypeptide and the sequence of SEQ ID NO: 1 or 6 based on the homology or identity between the sequences, for example using a BLAST algorithm.

In some embodiments, a polypeptide variant of the present invention may differ from SEQ ID NO: 1 or 6 by, for example, 1 to 50, 1 to 45, 1 to 40, 1 to 35, 1 to 30, 1 to 25, 1 to 20, 1 to 15, 1 to 10, 1 to 8, 1 to 6, 1 to 5, 1 to 4, e.g. 1, 2 or 3 amino acid substitutions, insertions and/or deletions, preferably 1 to 23, 1 to 20, 1 to 15, 1 to 10, 1 to 8, 1 to 6, 1 to 5, 1 to 4, e.g. 1, 2 to 3 amino acid substitutions and/or 1 to 32, 1 to 30, 1 to 25, 1 to 20, 1 to 15, 1 to 10, 1 to 8, 1 to 6, 1 to 5, 1 to 4, e.g. 1, 2 or 3 amino acid deletions. As discussed above, in some embodiments, it is preferred that deletions are at the N- and/or C-terminus, preferably the N-terminus, i.e. truncations, thereby generating polypeptide portions of SEQ ID NO: 1 or 6 as defined above, such as the portions disclosed in SEQ ID NOs: 2 and 7.

In some embodiments, any mutations that are present in the polypeptide of the present invention relative to the exemplified polypeptides (SEQ ID NO: 6 or 16) may be conservative amino acid substitutions. A conservative amino acid substitution refers to the replacement of an amino acid by another which preserves the physicochemical character of the polypeptide (e.g. D may be replaced by E or vice versa, N by Q, or L or I by V or vice versa). Thus, generally the substituting amino acid has similar properties, e.g. hydrophobicity, hydrophilicity, electronegativity, bulky side chains etc. to the amino acid being replaced. Isomers of the native L-amino acid e.g. D-amino acids may be incorporated.

In some embodiments, a cognate peptide tag variant as defined herein may differ from SEQ ID NOs: 3-5 by, for example, 1 to 5, 1 to 4, e.g. 1, 2 to 3 amino acid substitutions, insertions and/or deletions, preferably substitutions, as defined above.

In some embodiments, said cognate peptide tag sequence described herein is at least 80, 85, 90, 95, 96, 97, 98, 99 or 100% identical to the sequence (SEQ ID NOs: 3-5) to which it is compared.

In some embodiments, said polypeptide sequence above is at least 85, 90, 95, 96, 97, 98, 99 or 100% identical to the sequence (SEQ ID NOs: 1, 2, 6 or 7) to which it is compared.

Sequence identity may be determined by any suitable means known in the art, e.g. using the SWISS-PROT protein sequence databank using FASTA pep-cmp with a variable pamfactor, and gap creation penalty set at 12.0 and gap extension penalty set at 4.0, and a window of 2 amino acids. Other programs for determining amino acid sequence identity include the BestFit program of the Genetics Computer Group (GCG) Version 10 Software package from the University of Wisconsin. The program uses the local homology algorithm of Smith and Waterman with the default values: Gap creation penalty - 8, Gap extension penalty = 2, Average match = 2.912, Average mismatch = -2.003.

Preferably said comparison is made over the full length of the sequence, but may be made over a smaller window of comparison, e.g. less than 100, 80 or 50 contiguous amino acids.

Preferably the cognate peptide tag and polypeptide variants (e.g. sequence identity-related variants) are functionally equivalent to the cognate peptide tag and polypeptide having a sequence as set forth in SEQ ID NOs: 3-5 or SEQ ID NOs: 6, 7 or 16, respectively.

As referred to herein, "functional equivalence" refers to variants of the cognate peptide tag described herein and polypeptide of the invention discussed above that may show some reduced selectively (e.g. specificity) or affinity in the binding (formation of the non-covalent complex) with its respective partner (e.g. lower purity or yield in the process of the invention, or activity in a limited range of reaction conditions) relative to the parent molecule (i.e. the molecule with which it shows sequence homology), but preferably are as efficient or are more efficient.

A mutant or variant cognate peptide tag described herein with activity that is "equivalent" to the activity of a cognate peptide tag comprising or consisting of an amino acid sequence as set forth in one of SEQ ID NOs: 3-5 may have activity that is similar (i.e. comparable) to the activity of a peptide tag comprising or consisting of an amino acid sequence as set forth in one of SEQ ID NOs: 3-5, i.e. such that the practical applications of the peptide tag are not significantly affected, e.g. within a margin of experimental error.

Thus, an equivalent peptide tag activity means that the mutant or variant cognate peptide tag described is capable of binding selectively and reversibly to the polypeptide of the invention (particularly a polypeptide comprising an amino acid sequence as set forth in SEQ ID NO: 6, 7 or 16). In some preferred embodiments, the mutant or variant cognate peptide tag is capable of spontaneously forming an isopeptide bond with a polypeptide comprising or consisting of an amino acid sequence as set forth in SEQ ID NO: 11, 12 or 13 with a similar reaction rate (i.e. rate constant as discussed below) and/or yield to a peptide tag comprising or consisting of an amino acid sequence as set forth in one of SEQ ID NOs: 3-5 under the same conditions.

Similarly, a mutant or variant polypeptide of the invention with activity that is "equivalent" to the activity of a polypeptide comprising or consisting of an amino acid sequence as set forth in SEQ ID NO: 6, 7 or 16 (preferably SEQ ID NO: 6 or 16) may have activity that is similar (i.e. comparable) to the activity of a polypeptide comprising or consisting of an amino acid sequence as set forth in SEQ ID NO: 6, 7 or 16 (preferably SEQ ID NO: 6 or 16), i.e. such that the practical applications of the polypeptide are not significantly affected, e.g. within a margin of experimental error.

Thus, an equivalent polypeptide activity means that the mutant or variant polypeptide of the invention is capable of binding selectively and reversibly to the cognate peptide tag described herein (e.g. comprising or consisting of an amino acid sequence as set forth in one of SEQ ID NOs: 3-5) with a similar affinity and/or yield, as described above, to a polypeptide comprising or consisting of an amino acid sequence as set forth in SEQ ID NO: 6, 7 or 16 (preferably SEQ ID NO: 6 or 16) under the same conditions.

A mutant or variant polypeptide of the invention with activity that is "equivalent" to the activity of a polypeptide comprising or consisting of an amino acid sequence as set forth in SEQ ID NO: 6, 7 or 16 may compete with a polypeptide comprising or consisting of an amino acid sequence set forth in SEQ ID NO: 6 or 16 (SpySwitch) for binding with a cognate peptide tag as defined herein, e.g. one or all of SEQ ID NOs: 3-5.

The activity of different polypeptides (e.g. SEQ ID NO: 6 versus mutant) measured under the same reaction conditions, e.g. temperature, ligands (i.e. cognate peptide tag sequence) and their concentration, buffer, salt etc. as exemplified above, can be readily compared to determine whether the affinity and/or yield for each polypeptide is higher, lower or equivalent.

Hence, any modification or combination of modifications may be made to SEQ ID NO: 1 or 6 to produce a variant polypeptide of the invention (i.e. a polypeptide that satisfies the functional equivalence requirements defined above), provided that the variant polypeptide comprises an amino acid selected from alanine, glycine, serine, asparagine or threonine at a position equivalent to position 77 of SEQ ID NO: 1 and histidine at positions equivalent to positions 30, 84 and 85 of SEQ ID NO: 1. In some embodiments, the variant polypeptide comprises one or more (preferably all) of the following:
(1) glutamic acid at a position equivalent to position 91 of SEQ ID NO: 1;
(2) aspartic acid at a position equivalent to position 103 of SEQ ID NO: 1;
(3) histidine at a position equivalent to position 28 and/or 32 of SEQ ID NO: 1; and
(4) threonine at a position equivalent to position 59 of SEQ ID NO: 1;
(5) isoleucine at a position equivalent to position 94 of SEQ ID NO: 1;
(6) proline at a position equivalent to position 111 of SEQ ID NO: 1; and optionally at least one (preferably 2, 3, 4, 5, 6, 7, 8, 9, 10, 11, 12 or 13) other amino acid residue(s) at positions equivalent to positions 2, 9, 13, 19, 31, 37, 49, 62, 89, 97, 105, 108 and 113 of SEQ ID NO: 1 as defined above and retains the functional characteristics defined above, i.e. it results in a polypeptide that binds selectively and reversibly to a cognate peptide tag comprising or consisting of an amino acid sequence as set forth in one of SEQ ID NOs: 3-5 and optionally results in a process with an equivalent or higher purity and/or yield, temperature and/or pH range relative to a polypeptide having an amino acid sequence as set forth in SEQ ID NO: 6.

Alternatively viewed, any modification or combination of modifications (preferably substitutions) may be made to SEQ ID NO: 2 or 7 to produce a variant polypeptide of the invention (i.e. a polypeptide that satisfies the functional equivalence requirements defined above), provided that the variant polypeptide comprises an amino acid selected from alanine, glycine, serine, asparagine, or threonine at a position equivalent to position 56 and histidine at positions equivalent to positions 9, 63 and 64 of SEQ ID NO: 2. In some embodiments, the variant polypeptide comprises one or more (preferably all) of the following:
(1) glutamic acid at a position equivalent to position 70 of SEQ ID NO: 2;
(2) aspartic acid at a position equivalent to position 82 of SEQ ID NO: 2;
(3) histidine at a position equivalent to position 7 and/or 11 of SEQ ID NO: 2; and
(4) threonine at a position equivalent to position 38 of SEQ ID NO: 2;
(5) isoleucine at a position equivalent to position 73 of SEQ ID NO: 2;
(6) proline at a position equivalent to position 90 of SEQ ID NO: 2; and
and optionally at least one (preferably 2, 3, 4, 5, 6, 7, 8 or 9) other amino acid residue(s) at positions equivalent to positions 10, 16, 28, 41, 68, 76, 84, 87 and 92 of SEQ ID NO: 2 as defined above and retains the functional characteristics defined above, i.e. it results in a polypeptide that binds selectively and reversibly to a cognate peptide tag comprising or consisting of an amino acid sequence as set forth in one of SEQ ID NOs: 3-5 and optionally results in a process with an equivalent or higher purity and/or yield, temperature and/or pH range relative to a polypeptide having an amino acid sequence as set forth in SEQ ID NO: 6 or 7.

The terms "tag" and "peptide tag" as used herein generally refer to a peptide.

The term "peptide tag binding partner", "binding partner" or "catcher" as used herein generally refers to a polypeptide or protein.

In this respect, there is no standard definition regarding the size boundaries between what is meant by peptide or polypeptide. Typically a peptide may be viewed as comprising between 2-39 amino acids. Accordingly, a polypeptide may be viewed as comprising at least 40 amino acids, preferably at least 50, 60, 70, 80, 90, 100 or 110 amino acids.

Thus, in preferred embodiments a peptide tag as defined herein may be viewed as comprising at least 12 amino acids, e.g. 12-39 amino acids, such as e.g. 13-35, 14-34, 15-33, 16-31, 17-30 amino acids in length, e.g. it may comprise or consist of 12, 13, 14, 15, 16, 17, 18, 19 or 20 amino acids.

A polypeptide of the invention as defined herein may be viewed as comprising at least 80 amino acids, e.g. 80-150 amino acids, such as e.g. 80-140, 80-130, 80-120 amino acids in length, e.g. it may comprise or consist of 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 or 120 amino acids.

In this respect, it will be evident that the amino acid sequence set forth in SEQ ID NOs: 1 and 6 represents the core amino acid sequence of the polypeptide of the invention, which can contain additional sequences, particularly flanking sequences, e.g. peptide tags (e.g. a His-tag) and/or linkers. An example of an amino acid sequence of a polypeptide of the invention containing flanking sequences is provided in SEQ ID NO: 16. Thus, in some embodiments, the polypeptide of the invention comprises or consists of an amino acid sequence as set forth in SEQ ID NO: 16.

In a further embodiment, the invention provides a nucleic acid molecule comprising a nucleotide sequence which encodes a polypeptide of the invention as hereinbefore defined.

In some embodiments, the nucleic acid molecule encoding a polypeptide defined above comprises a nucleotide sequence as set forth in any one of SEQ ID NOs: 8, 9 or 17 or a nucleotide sequence with at least 80% sequence identity to a sequence as set forth in any one of SEQ ID NOs: 8, 9 or 17.

Preferably, the nucleic acid molecule above is at least 85, 90, 95, 96, 97, 98, 99 or 100% identical to the sequence to which it is compared.

Nucleic acid sequence identity may be determined by, e.g. FASTA Search using GCG packages, with default values and a variable pamfactor, and gap creation penalty set at 12.0 and gap extension penalty set at 4.0 with a window of 6 nucleotides. Preferably said comparison is made over the full length of the sequence, but may be made over a smaller window of comparison, e.g. less than 300, 200, 100 or 50 contiguous nucleotides.

The nucleic acid molecules of the invention may be made up of ribonucleotides and/or deoxyribonucleotides as well as synthetic residues, e.g. synthetic nucleotides, that are capable of participating in Watson-Crick type or analogous base pair interactions. Preferably, the nucleic acid molecule is DNA or RNA.

The nucleic acid molecules described above may be operatively linked to an expression control sequence, or a recombinant DNA cloning vehicle or vector containing such a recombinant DNA molecule. This allows cellular expression of the polypeptide of the invention as a gene product, the expression of which is directed by the gene(s) introduced into cells of interest. Gene expression is directed from a promoter active in the cells of interest and may be inserted in any form of linear or circular nucleic acid (e.g. DNA) vector for incorporation in the genome or for independent replication or transient transfection/expression. Suitable transformation or transfection techniques are well described in the literature. Alternatively, the naked nucleic acid (e.g. DNA or RNA, which may include one or more synthetic residues, e.g. base analogues) molecule may be introduced directly into the cell for the production of the polypeptide of the invention. Alternatively the nucleic acid may be converted to mRNA by *in vitro* transcription and the relevant protein may be generated by *in vitro* translation.

Appropriate expression vectors include appropriate control sequences such as, for example, translational (e.g. start and stop codons, ribosomal binding sites) and transcriptional control elements (e.g. promoter-operator regions, termination stop sequences) linked in matching reading frame with the nucleic acid molecules of the invention. Appropriate vectors may include plasmids and viruses (including both bacteriophage and eukaryotic viruses). Suitable viral vectors include baculovirus and also adenovirus, adeno-associated virus, herpes and vaccinia/pox viruses. Many other viral vectors are described in the art. Examples of suitable plasmid vectors include bacterial and mammalian expression vectors, pET vectors (e.g. pET28 vectors), pDEST, pGEX-KG, pEF-neo and pEF-HA.

In some embodiments, the polypeptide of the invention may comprise additional sequences (e.g. peptide/polypeptide tags to facilitate purification of the polypeptide prior to use in the process and use of the invention discussed below). Any suitable purification moiety or tag may be incorporated into the polypeptide and such moieties are well known in the art. For instance, in some embodiments, the polypeptide may comprise a peptide purification tag or moiety, e.g. a His-tag sequence. Such purification moieties or tags may be incorporated at any position within the polypeptide. In some preferred embodiments, the purification moiety is located at or towards (i.e. within 5, 10, 15, 20 amino acids of) the N- or C-terminus of the polypeptide.)

In some embodiments, a peptide tag (e.g. His-tag) is connected to the polypeptide of the invention via a spacer sequence. Thus, peptide tag and polypeptide of the invention may be linked directly to each other or they may be linked indirectly by means of one or more spacer sequences. Thus, a spacer sequence may interspace or separate two or more individual parts of the polypeptide of the invention. In some embodiments, a spacer may be N-terminal or C-terminal to the peptide tag or polypeptide of the invention. In some embodiments, spacers may be at both sides of the peptide tag or polypeptide of the invention.

The spacer sequence may be of variable length and/or sequence, for example it may have 1-40, more particularly 2-20, 1-15, 1-12, 1-10, 1-8, or 1-6 residues, e.g. 6, 7, 8, 9, 10 or more residues. By way of representative example the spacer sequence, if present, may have 1-15, 1-12, 1-10, 1-8 or 1-6 residues etc. The nature of the residues is not critical and they may for example be any amino acid, e.g. a neutral amino acid, or an aliphatic amino acid, or alternatively they may be hydrophobic, or polar or charged or structure-forming e.g. proline. In some preferred embodiments, the linker is a serine and/or glycine-rich sequence, such as GSGGSG (SEQ ID NO: 18) or GSSGS (SEQ ID NO: 19).

By way of example, a polypeptide of the invention comprising a peptide tag (His-tag) and spacer sequences is set forth in SEQ ID NO: 16.

Accordingly, the nucleic acid molecule may conveniently be fused with DNA encoding an additional peptide or polypeptide, e.g. His-tag, maltose-binding protein, to produce a fusion protein on expression. The nucleotide sequence of a nucleic acid molecule encoding a polypeptide of the invention comprising a peptide tag (His-tag) and spacer sequences is set forth in SEQ ID NO: 17.

Thus viewed from a further aspect, the present invention provides a vector, preferably an expression vector, comprising a nucleic acid molecule as defined above.

Other aspects of the invention include methods for preparing recombinant nucleic acid molecules according to the invention, comprising inserting nucleic acid molecule of the invention encoding the polypeptide of the invention into vector nucleic acid.

Nucleic acid molecules of the invention, preferably contained in a vector, may be introduced into a cell by any appropriate means. Suitable transformation or transfection techniques are well described in the literature. Numerous techniques are known and may be used to introduce such vectors into prokaryotic or eukaryotic cells for expression. Preferred host cells for this purpose include insect cell lines, yeast, mammalian cell lines or *Escherichia coli,* such as strain BL21/DE3 or C41(DE3). The invention also extends to transformed or transfected prokaryotic or eukaryotic host cells containing a nucleic acid molecule, particularly a vector as defined above.

Thus, in another aspect, there is provided a recombinant host cell containing a nucleic acid molecule and/or vector as described above.

By "recombinant" is meant that the nucleic acid molecule and/or vector has been introduced into the host cell. The host cell may or may not naturally contain an endogenous copy of the nucleic acid molecule, but it is recombinant in that an exogenous or further endogenous copy of the nucleic acid molecule and/or vector has been introduced.

A further aspect of the invention provides a method of preparing a polypeptide of the invention as hereinbefore defined, which comprises culturing a host cell containing a nucleic acid molecule as defined above, under conditions whereby said nucleic acid molecule encoding said polypeptide is expressed and recovering said polypeptide thus produced. The expressed polypeptide forms a further aspect of the invention.

In some embodiments, the polypeptide of the invention, or for use in the processes and uses of the invention, may be generated synthetically, e.g. by ligation of amino acids or smaller synthetically generated peptides, or more conveniently by recombinant expression of a nucleic acid molecule encoding said polypeptide as described hereinbefore.

Nucleic acid molecules of the invention may be generated synthetically by any suitable means known in the art.

Thus, the polypeptide of the invention may be an isolated, purified, recombinant or synthesised polypeptide.

The term "polypeptide" is used herein interchangeably with the term "protein". As noted above, the term polypeptide or protein typically includes any amino acid sequence comprising at least 40 consecutive amino acid residues, e.g. at least 50, 60, 70, 80, 90, 100, 150 amino acids, such as 40-1000, 50-900, 60-800, 70-700, 80-600, 90-500, 100-400 amino acids.

Standard amino acid nomenclature is used herein. Thus, the full name of an amino acid residue may be used interchangeably with one letter code or three letter abbreviations. For instance, lysine may be substituted with K or Lys, isoleucine may be substituted with I or Ile, and so on. Moreover, the terms aspartate and aspartic acid, and glutamate and glutamic acid are used interchangeably herein and may be replaced with Asp or D, or Glu or E, respectively.

As discussed above, the polypeptide of the present invention forms one part of a two-part affinity purification system and finds particular utility in purifying (i.e. isolating or separating) molecules or components (fusion partners) comprising (e.g. joined or conjugated to) a cognate peptide tag as defined herein.

Thus, in a further aspect, the invention may be seen to provide the use of a polypeptide of the invention defined above to purify or isolate a molecule or component comprising a cognate peptide tag as defined herein, e.g. a peptide tag having an amino acid sequence with at least 80% sequence identity to a sequence as set forth in one of SEQ ID NOs: 3-5, preferably wherein said peptide comprises an aspartic acid at a position equivalent to position 7 of SEQ ID NO: 3, position 8 of SEQ ID NO: 4 and position 10 of SEQ ID NO: 5.

Affinity purification systems typically utilise a capture molecule (e.g. receptor) immobilised on a solid substrate to facilitate the capture, washing and elution of the target ligand. Thus, the polypeptide of the invention may be immobilised (e.g. fused, conjugated or linked) to a solid substrate (i.e. a solid phase or solid support). It will be evident that this may be achieved in any convenient way. Alternatively viewed, the invention provides a solid support (e.g. resin and/or beads) on which the polypeptide of the invention is immobilised.

The manner or means of immobilisation and the solid support may be selected, according to choice, from any number of immobilisation means and solid supports as are widely known in the art and described in the literature. Thus, the polypeptide of the invention may be directly bound to the support, for example via a domain or moiety of the polypeptide (e.g. chemically cross-linked). In some embodiments, the polypeptide may be bound indirectly by means of a linker group, or by an intermediary binding group(s) (e.g. by means of a biotin-streptavidin interaction). Thus, the polypeptide may be covalently or non-covalently linked to the solid support. In certain embodiments the polypeptide is immobilised on a solid substrate via a covalent bond.

The linkage may be a reversible (e.g. cleavable) or irreversible linkage. Thus, in some embodiments, the linkage may be cleaved enzymatically, chemically, or with light, e.g. the linkage may be a light-sensitive linkage.

Linking groups of interest may vary widely depending on the nature of the solid support. The linking group, when present, is in many embodiments biologically inert.

Many linking groups are known to those of skill in the art and find use in the invention. In representative embodiments, the linking group is generally at least about 50 daltons, usually at least about 100 daltons and may be as large as 1000 daltons or larger, for example up to 1000000 daltons if the linking group contains a spacer, but generally will not exceed about 500 daltons and usually will not exceed about 300 daltons. Generally, such linkers will comprise a spacer group terminated at either end with a reactive functionality capable of covalently bonding to the solid support.

Spacer groups of interest may include aliphatic and unsaturated hydrocarbon chains, spacers containing heteroatoms such as oxygen (ethers such as polyethylene glycol) or nitrogen (polyamines), peptides, carbohydrates, cyclic or acyclic systems that may possibly contain heteroatoms. Spacer groups may also be comprised of ligands that bind to metals such that the presence of a metal ion coordinates two or more ligands to form a complex. Specific spacer elements include: 1,4-diaminohexane, xylylenediamine, terephthalic acid, 3,6-dioxaoctanedioic acid, ethylenediamine-N,N-diacetic acid, 1,1'-ethylenebis(5-oxo-3-pyrrolidinecarboxylic acid), 4,4'-ethylenedipiperidine, oligoethylene glycol and polyethylene glycol. Potential reactive functionalities include nucleophilic functional groups (amines, alcohols, thiols, hydrazides), electrophilic functional groups (aldehydes, esters, vinyl ketones, epoxides, isocyanates, maleimides), functional groups capable of cycloaddition reactions, forming disulfide bonds, or binding to metals. Specific examples include primary and secondary amines, hydroxamic acids, N-hydroxysuccinimidyl esters, N-hydroxysuccinimidyl carbonates, oxycarbonylimidazoles, nitrophenylesters, trifluoroethyl esters, glycidyl ethers, vinylsulfones, and maleimides. Specific linker groups that may find use in coupling the polypeptide of the invention to a solid substrate include heterofunctional compounds, such as azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, and succinimidyl-4-[N-maleimidomethyl]cyclohexane-1-carboxylate, 3-(2-pyridyldithio)propionic acid N-hydroxysuccinimide ester (SPDP), 4-(N-maleimidomethyl)-cyclohexane-1-carboxylic acid N-hydroxysuccinimide ester (SMCC), and the like. For instance, a spacer may be formed with an azide reacting with an alkyne or formed with a tetrazine reacting with a trans-cyclooctene or a norbornene.

In some embodiments, a polypeptide may be provided with means for immobilisation (e.g. an affinity binding partner, e.g. biotin or a hapten, capable of binding to its binding partner, i.e. a cognate binding partner, e.g. streptavidin or an antibody) provided on the support. In some embodiments, the interaction between the polypeptide and the solid support must be robust enough to allow for washing and elution steps in the process defined below, i.e. the interaction between the polypeptide and solid support is not disrupted (significantly disrupted) by the washing or elution steps. For instance, it is preferred that with each washing and/or elution step, less than 5%, preferably less than 4, 3, 2, 1, 0.5 or 0.1% of the polypeptide of the invention is removed or eluted from the solid phase.

As mentioned above and discussed in detail in the Examples, the inventors have determined that the cysteine at position 49 of the polypeptide of the invention facilitates the coupling of the polypeptide to a solid support with minimal disruption to SpyTag peptide binding and elution. Without wishing to be bound by theory, it is hypothesised that coupling the polypeptide of the invention to a solid support via the cysteine residue at position 49 of SEQ ID NO: 1 maximised accessibility of the polypeptide to its cognate peptide tag (i.e. SpyTag and variants thereof).

Thus, in some embodiments the polypeptide is immobilised on a solid substrate via a covalent bond between the cysteine at a position equivalent to position 49 of SEQ ID NO: 1. In a particular embodiment, the polypeptide comprises an amino acid sequence with at least 80% sequence identity to a sequence as set forth in SEQ ID NO: 1, wherein the polypeptide comprises alanine, glycine, serine, asparagine or threonine at a position equivalent to position 77 of SEQ ID NO: 1, histidine at positions equivalent to positions 30, 84 and 85 of SEQ ID NO: 1 and cysteine at a position equivalent to position 49 of SEQ ID NO: 1, wherein the polypeptide binds selectively and reversibly to a peptide comprising an amino acid sequence as set forth in SEQ ID NO: 3, 4 or 5 and wherein the polypeptide is immobilised on a solid substrate via a covalent bond between said cysteine and the solid substrate. The covalent bond may be formed by reacting the thiol group on the cysteine residue with an appropriate reactive group on the solid substrate. Thus, in some embodiments, the solid substrate may comprise an iodoacetyl group, e.g. the solid substrate may be an iodoacetyl-activated substrate.

Whilst it may be advantageous to immobilise the polypeptide of the invention on a solid support via the cysteine residue at position 49 of SEQ ID NO: 1 or 6, this is not essential.

The solid support (phase or substrate) may be any of the well-known supports or matrices which are currently widely used or proposed for immobilisation, separation etc. These may take the form of particles (e.g. beads which may be magnetic, para-magnetic or non-magnetic), sheets, gels, filters, membranes, fibres, capillaries, slides, arrays or microtitre strips, tubes, plates or wells etc.

The support may be made of glass, silica, latex or a polymeric material, e.g. polyacrylamide or a polysaccharide polymer material, such as agarose (e.g. sepharose) or dextran. Suitable are materials presenting a high surface area for binding of the polypeptide. Such supports may have an irregular surface and may be for example porous or particulate, e.g. particles, fibres, webs, sinters or sieves. Particulate materials, e.g. beads, are useful due to their greater binding capacity, particularly polymeric (e.g. agarose) beads.

Conveniently, a particulate solid support used according to the invention will comprise spherical beads. The size of the beads is not critical, but they may for example be of the order of diameter of at least about 1 µm and preferably at least about 2 µm, 5 µm, 10 µm or 20 µm and have a maximum diameter of preferably not more than about 500 µm, and e.g. not more than about 100 µm.

Monodisperse particles, that is those which are substantially uniform in size (e.g. size having a diameter standard deviation of less than 5%) have the advantage that they provide very uniform reproducibility of reaction. Representative monodisperse polymer particles may be produced by the technique described in US-A-4336173.

However, to aid manipulation and separation, magnetic beads are advantageous. The term "magnetic" as used herein means that the support is capable of having a magnetic moment imparted to it when placed in a magnetic field, and thus is displaceable under the action of that field. In other words, a support comprising magnetic particles may readily be removed by magnetic aggregation, which provides a quick, simple and efficient way of separating the particles following the binding to the SpyTag variant.

In some embodiments, the solid support is a resin, preferably a thiol-reactive resin. As described in the Examples, the inventors have determined that the polypeptide advantageously may be stored under suitable conditions for long periods, e.g. weeks, months or years, without significant loss of activity. Thus, in some embodiments, it is preferred that the resin is also stable in long-term storage under suitable conditions, such as in 20% ethanol at temperatures between 0-10 °C, such as about 4 °C.

Whilst it is envisaged that the polypeptide of, and for use in, the invention may be produced recombinantly, and this is a preferred embodiment of the invention, it will be evident that it may be useful to modify one or more residues in the polypeptide to facilitate its immobilisation on a solid substrate and/or to improve the stability of the polypeptide. Thus, in some embodiments, the polypeptide of, or for use in, the invention may comprise unnatural or non-standard amino acids.

In some embodiments, the polypeptide of, or for use in, the invention may comprise one or more, e.g. at least 1, 2, 3, 4, 5 non-conventional amino acids, such as 10, 15, 20 or more non-conventional amino acids, i.e. amino acids which possess a side chain that is not coded for by the standard genetic code, termed herein "non-coded amino acids". Such amino acids are well known in the art, and may be selected from amino acids which are formed through metabolic processes such as ornithine or taurine, and/or artificially modified amino acids such as 9H-fluoren-9-ylmethoxycarbonyl (Fmoc), (tert)-(B)utyl (o)xy (c)arbonyl (Boc), 2,2,5,7,8-pentamethylchroman-6-sulphonyl (Pmc) protected amino acids, or amino acids having the benzyloxy-carbonyl (Z) group.

Examples of non-standard or structural analogue amino acids which may be used in the polypeptide of, and for use in, the invention are D amino acids, amide isosteres (such as N-methyl amide, retro-inverse amide, thioamide, thioester, phosphonate, ketomethylene, hydroxymethylene, fluorovinyl, (E)-vinyl, methyleneamino, methylenethio or alkane), L-N methylamino acids, D-α methylamino acids, D-N-methylamino acids. Further non-standard amino acids which may be used in the polypeptide of, and for use in, the invention are disclosed in Willis and Chin, Nat Chem. 2018; 10(8):831-837, in Table 1 of WO2018/189517 and WO2018/197854.

Thus, in a further aspect the invention provides a process for purifying or isolating a molecule or component comprising a peptide (i.e. a cognate peptide tag) having an amino acid sequence with at least 80% sequence identity to a sequence as set forth in SEQ ID NO: 3, 4 or 5, wherein said peptide comprises an aspartic acid at a position equivalent to position 7 of SEQ ID NO: 3, position 8 of SEQ ID NO: 4 or position 10 of SEQ ID NO: 5, said process comprising:
a) providing a solid substrate on which a polypeptide of the invention is immobilised;
b) providing a sample comprising said molecule or component;
c) contacting the solid substrate of a) with the sample of b) under conditions that enable said peptide to selectively bind to said polypeptide, thereby forming a non-covalent complex between said polypeptide immobilised on the solid substrate and molecule or component comprising said peptide;
d) washing the solid substrate with a wash solution;
e) separating the molecule or component comprising the peptide from the polypeptide immobilised on the solid substrate.

The cognate peptide tag of the affinity purification system described herein may be fused or conjugated to other molecules or to other components or entities (i.e. fusion partners) to facilitate their purification prior to other downstream applications, e.g. reacting the cognate peptide tag with a SpyCatcher polypeptide (such as a polypeptide comprising an amino acid sequence as set forth in SEQ ID NO: 11, 12 or 13). Such molecules or components (i.e. entities) may be a nucleic acid molecule, protein (e.g. an antibody), peptide, small-molecule organic compound, fluorophore, metal-ligand complex, polysaccharide, nanoparticle, nanotube, polymer, cell, organelle, vesicle, virus, virus-like particle or any combination of these.

Thus, process or use of the invention may be used for the purification or isolation of a nucleic acid molecule, protein (e.g. an antibody), peptide, small-molecule organic compound, fluorophore, metal-ligand complex, polysaccharide, nanoparticle, nanotube, polymer, cell, organelle, vesicle, virus, virus-like particle or any combination of these to which the cognate peptide tag is fused or conjugated.

The cell may be a prokaryotic or eukaryotic cell.

In some embodiments, the cognate peptide tag may be conjugated or fused to a compound or molecule which has a therapeutic or prophylactic effect, e.g. an antibiotic, antiviral, vaccine, antitumour agent, e.g. a radioactive compound or isotope, cytokines, toxins, oligonucleotides and nucleic acids encoding genes or nucleic acid vaccines.

In some embodiments, the cognate peptide tag may be conjugated or fused to a label, e.g. a radiolabel, a fluorescent label, luminescent label, a chromophore label as well as to substances and enzymes which generate a detectable substrate, e.g. horseradish peroxidase, luciferase or alkaline phosphatase. This detection may be applied in numerous assays where antibodies are conventionally used, including Western blotting/immunoblotting, histochemistry, enzyme-linked immunosorbent assay (ELISA), or flow cytometry (FACS) formats. Labels for magnetic resonance imaging, positron emission tomography probes and boron 10 for neutron capture therapy may also be conjugated to the peptide tag described herein. Particularly, the peptide tag may be fused or produced with another peptide and/or may be fused or produced with another protein.

In a particularly useful embodiment, the cognate peptide tag is fused or conjugated with another peptide or polypeptide. For instance, the cognate peptide tag may be produced as part of another peptide or polypeptide using recombinant techniques as discussed above, i.e. as a recombinant or synthetic protein or polypeptide. Thus, in some embodiments, the process or use of the invention may be used for the purification or isolation of a recombinant or synthetic protein or polypeptide comprising the cognate peptide tag described herein.

It will be evident that the cognate peptide tag defined herein may be fused to any protein or polypeptide. The protein may be derived or obtained from any suitable source. For instance, the protein may be *in vitro* translated or purified from biological and clinical samples, e.g. any cell or tissue sample of an organism (eukaryotic, prokaryotic), or any body fluid or preparation derived therefrom, as well as samples such as cell cultures, cell preparations, cell lysates etc. Proteins may be derived or obtained, e.g. purified from environmental samples, e.g. soil and water samples or food samples are also included. The samples may be freshly prepared or they may be prior-treated in any convenient way e.g. for storage.

As noted above, in a preferred embodiment, the protein may be produced recombinantly and thus the nucleic acid molecules encoding said proteins may be derived or obtained from any suitable source, e.g. any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including bluegreen algae, fungi, bacteria, protozoa, viruses etc. In some embodiments, the proteins may be synthetic proteins. For example, the peptide and polypeptide (proteins) disclosed herein may be produced by chemical synthesis, such as solidphase peptide synthesis.

The terms "conjugating" or "linking" in the context of the present invention with respect to connecting the cognate peptide tag to molecules or components for purification or isolation in the process or use of the invention refers to joining said peptide tag to said molecules or components, e.g. proteins, via a covalent bond, particularly a peptide bond between the peptide tag and a polypeptide. With respect to connecting the polypeptide of the invention to a solid substrate, "conjugating" or "linking" refers to joining said polypeptide to said solid substrate, e.g. beads, via a covalent bond, particularly a thioether bond between the polypeptide and solid substrate.

The sample used in the process of the invention (i.e. comprising the molecule or component comprising the cognate peptide tag, e.g. recombinant protein) may be from any biological, laboratory or clinical sample, e.g. any cell or tissue sample of an organism (eukaryotic, prokaryotic), or any body fluid or preparation derived therefrom, as well as samples such as cell cultures, cell preparations, cell lysates etc. The samples may be freshly prepared or they may be prior-treated in any convenient way, e.g. for storage.

As noted above, the non-covalent interaction between the polypeptide of the invention and its cognate peptide tag is stable under specific conditions, i.e. neutral pH and low to ambient temperatures (e.g. 0-25 °C) and may be disrupted by altering the pH and/or temperature conditions.

Thus, in some embodiments, the step of contacting the solid substrate of a) with the sample of b) occurs under the conditions of:
i) a temperature of about 0-25 °C, preferably about 0-15 or about 0-10 °C; and
ii) a neutral pH, preferably a pH of about 6.5-8.5, preferably about 7.0-8.0 or about 7.3-7.7.

Alternatively viewed, conditions that enable said peptide to selectively bind to said polypeptide include:
i) a temperature of about 0-25 °C, preferably about 0-15 or about 0-10 °C; and
ii) a neutral pH, preferably a pH of about 6.5-8.5, preferably about 7.0-8.0 or about 7.3-7.7.

It will be evident that the specific combination of temperature and pH conditions may be selected from those outlined above to ensure they are suitable for the molecule or component comprising the peptide tag. However, particularly preferred conditions for the step of contacting the solid substrate of a) with the sample of b) include a pH of about 7.3-7.7 (e.g. about 7.5) and a temperature of about 0-10 °C (e.g. about 4-8 °C).

In some embodiments, the step of separating the molecule or component comprising the peptide from the polypeptide immobilised on the solid substrate may comprise subjecting the solid substrate to conditions suitable to disrupt the polypeptide:cognate peptide tag complex, i.e. to disrupt the non-covalent interaction between the polypeptide and the cognate peptide tag.

In some embodiments, conditions suitable to disrupt the polypeptide:cognate peptide tag complex comprise contacting said complex with a with an acidic solution, e.g. a solution with a pH of about 3.0-6.0, preferably about 3.5-5.5 or 4.0-5.0, e.g. about pH 5.0.). Alternatively or additionally, the step of separating the molecule or component comprising the peptide may comprise elevating or increasing the temperature of the non-covalent complex between said polypeptide immobilised on the solid substrate and molecule or component comprising said peptide to at least about 25 °C, such as at least about 30 °C or 35 °C, e.g. about 25-45 °C or 30-40 °C. In some embodiments, conditions suitable to disrupt the polypeptide:cognate peptide tag complex comprise incubating said complex with a solution comprising a competitor (e.g. 100 µM to 2 mM of the cognate peptide tag as defined above (most preferably SEQ ID NO: 5)).

In some embodiments, the solid substrate may be subjected to these conditions repeatedly, e.g. 2, 3, 4, 5 or more times, in order to maximise the yield of the molecule or component to be purified. In some embodiments, it may be advantageous to use a combination of conditions to maximise the yield of the molecule or component to be purified, e.g. a first step using an acidic solution and a second step using an elevated temperature. In some embodiments, the combination of conditions may be applied simultaneously, e.g. contacting the complex with an acidic solution at an elevated temperature, and this combined step may be repeated as defined above. Any suitable combination of conditions may be used and is within the purview of the skilled person. In embodiments where competitive peptide elution is used, i.e. wherein the complex is incubated with a competitor, such as the cognate peptide tag, the elution step may be repeated multiple times, e.g. 2, 3, 4, 5 or more times.

Thus, in some embodiments, the step of separating the molecule or component comprising the peptide comprises:
(i) contacting said complex with an acidic solution, optionally a buffered solution with a pH of about 3.0-6.0, preferably about 3.5-5.5; and/or
(ii) increasing the temperature of said complex to at least about 25 °C, 30 °C or 35 °C, preferably about 25-45 °C, 30-40 °C or 35-45 °C, optionally comprising contacting said complex with a solution (e.g. an acidic solution) having a temperature of at least about 25 °C, 30 °C or 35 °C, preferably about 25-45 °C, 30-40 °C or 35-45 °C; and/or
(iii) contacting said complex with a solution comprising a peptide comprising or consisting of an amino acid sequence with at least 80% sequence identity to an amino acid sequence as set forth in one of SEQ ID NOs: 3-5, preferably a peptide comprising or consisting of an amino acid sequence as set forth in one of SEQ ID NOs: 3-5.

In some embodiments, it is preferred that the step of separating the molecule or component comprising the peptide by increasing the temperature of the complex is performed at a neutral pH as defined herein, e.g. about 6.5-8.5.

Preferably the conditions that are used to disrupt the polypeptide:cognate peptide tag complex are such that the cognate peptide tag can still be used in downstream applications, i.e. the conditions do not lead to irreversible loss of activity of the cognate peptide tag.

While the use of SpyTag peptides for affinity purification is particularly advantageous because it provides the purified molecule or component with downstream functionality (i.e. the ability to be conjugated to other molecules via SpyCatcher polypeptides, e.g. SEQ ID NOs: 11, 12 and 13), the process of invention may find utility in the purification or isolation of only the target molecule or component, i.e. without the peptide tag. This may be achieved by separating the target molecule or component from the polypeptide immobilised on the solid substrate through a cleavage reaction that cleaves the peptide tag from the target molecule or component.

Thus, in some embodiments, the step of separating the molecule or component comprising the peptide from the polypeptide immobilised on the solid substrate may comprise subjecting the solid substrate to conditions suitable to cleave the peptide tag from the molecule or component comprising the peptide tag, i.e. by on-resin tag cleavage. This may be accomplished by incorporating (e.g. genetically encoding) a cleavage site which can be recognised by one or more proteases specific for that site between the peptide tag and the target molecule or component. Cleavage of the target molecule:peptide tag fusion at the cleavage site by the specific protease(s) releases the target molecule or component from the polypeptide:cognate peptide tag complex, leaving the peptide tag still bound to the polypeptide. Suitable proteases and their respective recognition sites are well known in the art, and any appropriate setup may be utilised in the present method.

Thus, in some embodiments, the molecule or component comprising the peptide tag contains a cleavage site between the peptide tag and molecule or component, e.g. a cleavage site linking the peptide tag and molecule or component. Alternatively viewed, the peptide tag is fused or conjugated to the molecule or component indirectly via a cleavable linker. In some embodiments, the cleavage site or cleavable linker is a protease cleavage site, such as a TEV recognition site (e.g. SEQ ID NO: 14). Thus, in some embodiments, the step of separating the molecule or component comprising the peptide from the polypeptide immobilised on the solid substrate may comprise contacting the solid substrate with an entity (e.g. protease, e.g. TEV protease) under conditions suitable to cleave the cleavage site or cleavable linker, thereby releasing the molecule or component from the peptide tag and the polypeptide immobilised on the solid substrate.

The step of washing the solid substrate with a wash solution prior to separating said molecule or component from the solid substrate may utilise any conditions suitable to maintain the non-covalent interaction between the polypeptide and peptide tag, e.g. conditions used in the contacting step. Thus, in some embodiments, the wash solution may be a buffered solution with a neutral pH, e.g. a pH of about 6.5-8.5, preferably about 7.0-8.0 or about 7.3-7.7 (e.g. about 7.5). For instance, the wash solution may comprise 20-100 mM (e.g. 50 mM) Tris-HCl pH 7.5 and about 100-500 mM (e.g. about 300 mM) NaCl.

The wash solution may be selected based on the molecules or components conjugated or linked to the peptide tag. Furthermore, the step of washing the solid substrate may be repeated multiple times, e.g. 2, 3, 4, 5 or more times. Alternatively viewed, in some embodiments the process comprises multiple wash steps, wherein the same or different washing conditions may be used in each step.

Where the solid substrate comprises beads (e.g. agarose-based beads) the volume of buffer used in the wash steps may be at least about 2 times the volume of the beads, e.g. at least about 3, 4, 5, 6, 7, 8, 9 or 10 times the volume of the beads.

In some embodiments, the solid substrate is subjected to stringent washing conditions. The nature of the stringent washing conditions will depend on the molecules or components conjugated or linked to the peptide tag and/or the composition of the solid substrate. The skilled person could select such conditions as a matter of routine.

The washing steps should be conducted at a temperature that minimises elution of the molecule or component comprising the peptide tag from the solid substrate, i.e. below a temperature used in the elution/separating step. A suitable "non-eluting" temperature may be determined readily by a person of skill in the art based on routine experimentation and may depend on the nature of the molecule or component being isolated or purified. In some embodiments, the washing steps are performed at about 10 °C or less, e.g. 9, 8, 7, 6, 5 or 4 °C or less.

Whilst it may be useful to immobilise the polypeptide of the invention on a solid support prior to contact with the sample comprising the molecule or component comprising the cognate peptide tag, it will be evident that this is not essential. For instance, the binding of the polypeptide of the invention and the component comprising the cognate peptide tag may take place in solution, which is subsequently applied to a solid support or solid phase, e.g. column, for subsequent washing and separation (e.g. elution) steps. In some embodiments, the polypeptide:cognate peptide tag complex may be applied to the solid phase under conditions suitable to immobilise the complex on the solid phase via the polypeptide (e.g. an immobilisation domain on the polypeptide), washed under suitable conditions and subsequently subjected to one or more of the conditions mentioned above, e.g. contacted with an acidic solution, to disrupt the complex, thereby separating the polypeptide and the component comprising the cognate peptide tag.

As described in the Examples below, the inventors have shown that once the molecule or component comprising the cognate peptide tag has been separated from the immobilised polypeptide, the solid support comprising the polypeptide may be reused, e.g. for further purification reactions. In some embodiments, the solid support comprising the polypeptide may be subject to a regeneration process, to ensure that all of the component or molecule comprising the peptide tag has been removed, before it is reused. This regeneration process may involve repeated washes under conditions suitable to remove any residual peptide tags and/or peptide tag complexes. For example, the solid support comprising the polypeptide may be subjected to washes of a low pH solution or buffer, a solution comprising a high concentration of urea (e.g. about 8 M) and/or NaOH. In some embodiments, the solid support comprising the polypeptide may be subjected to consecutive treatments of about 0.1 M glycine-HCl pH 2.0, about 8 M urea and about 0.1 M NaOH, optionally with a physiological wash (e.g. using a wash solution as described above) in between each treatment condition. In some embodiments, the regeneration process is performed at an elevated temperature, e.g. about 25 °C, 30 °C, 35 °C or more, such as 25-45 °C, 30-40 °C or 35-45 °C.

A "low pH solution or buffer" may be viewed as any solution or buffer suitable for disrupting the non-covalent interaction between the polypeptide of the invention and its cognate peptide tag partner. In some embodiments, the low pH solution or buffer is an antibody elution buffer. By way of example, antibody elution buffers may comprise or consist of 50-100 mM glycine pH 2.0-2.5. Thus, in some embodiments, the low pH solution or buffer has a pH of about 3.0 or less, e.g. about 1.5-3.0 or 2.0-3.0.

In a further aspect, the invention provides an apparatus for use in the process or use hereinbefore defined comprising a solid substrate on which a polypeptide of the invention is immobilised.

In some embodiments, the apparatus may comprise a chromatography column comprising the solid substrate on which a polypeptide of the invention is immobilised. The apparatus may further comprise means for contacting the solid substrate with the sample, washing and elution buffers/solutions and/or means for removing (e.g. aspirating) or collecting liquids (e.g. wash-through, eluted fractions) from the solid substrate.

In a further aspect, the invention provides a kit, particularly a kit for use in preparing a solid substrate on which a polypeptide of the invention is immobilised, comprising:
a) a polypeptide of the invention; and
b) means for immobilising the polypeptide of a) on a solid substrate.

In a further embodiment, the kit further comprises a solid substrate as defined above, e.g. resin and/or beads (e.g. agarose beads).

Means for immobilising the polypeptide of the invention on a solid substrate may comprise reagents for activating the solid substrate (e.g. resin) and/or polypeptide (e.g. tris(2-carboxyethyl)phosphine), reagents for coupling the polypeptide to the solid substrate (e.g. coupling buffer, such as 50 mM Tris-HCl, 5 mM EDTA, pH 8.5) and/or reagents for blocking the solid substrate (e.g. L-cysteine-HCl in coupling buffer).

The invention will now be described in more detail in the following nonlimiting Examples with reference to the following drawings:

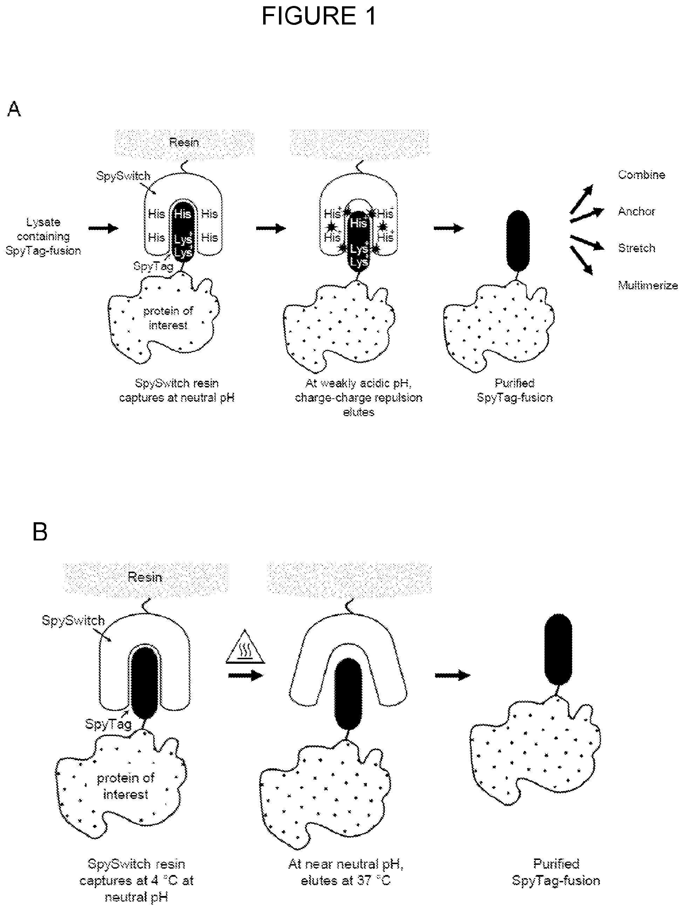

Figure 1: Overview of SpySwitch elution. Figure 1A depicts SpySwitch pH-dependent elution whereby SpyTag interacts with SpySwitch at neutral pH and is eluted at weakly acidic pH through charge-charge repulsion (represented by stars). Figure 1B depicts SpySwitch temperature-dependent elution whereby SpyTag interacts with SpySwitch at 4 °C and is eluted at 37 °C.

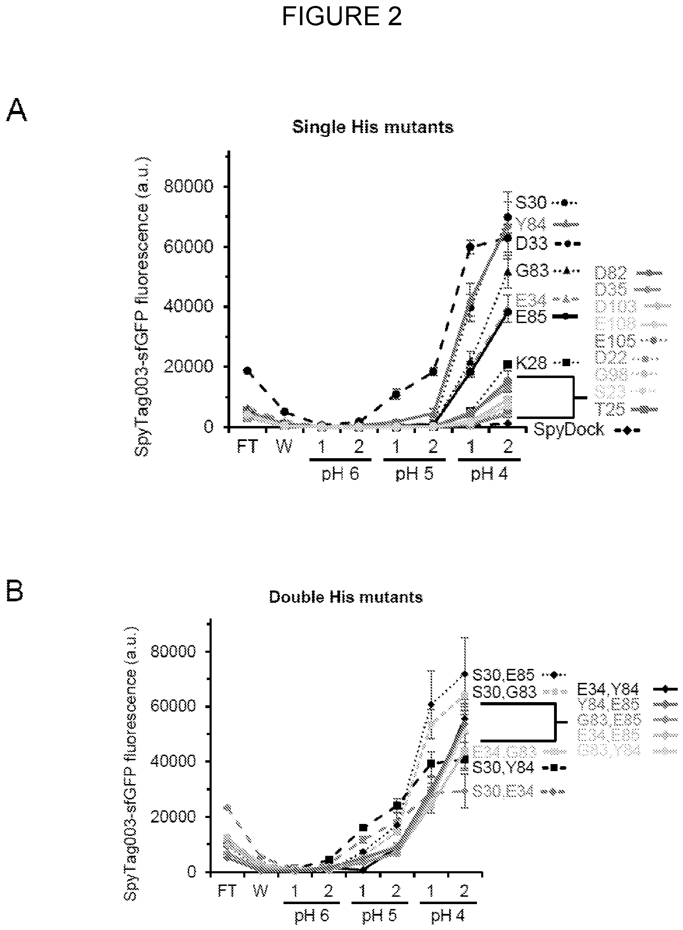

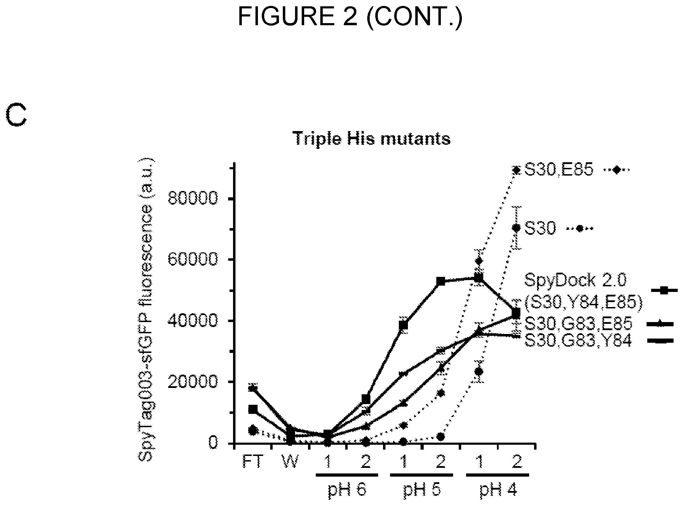

Figure 2: (A) Single histidine variants, (B) Double histidine variants and (C) Triple histidine mutants of SpyDock were assayed for SpyTag003-sfGFP binding and stepwise pH elution from pH 6.0 to pH 4.0. Each fraction was neutralised before fluorescence detection of SpyTag003-sfGFP. FT, flow-through; W, wash. Triple mutants are compared to the best single and double histidine mutant in Figure 2C. The mean is represented by filled shapes, with the error bars ± 1 s.d.; a.u.: arbitrary units.

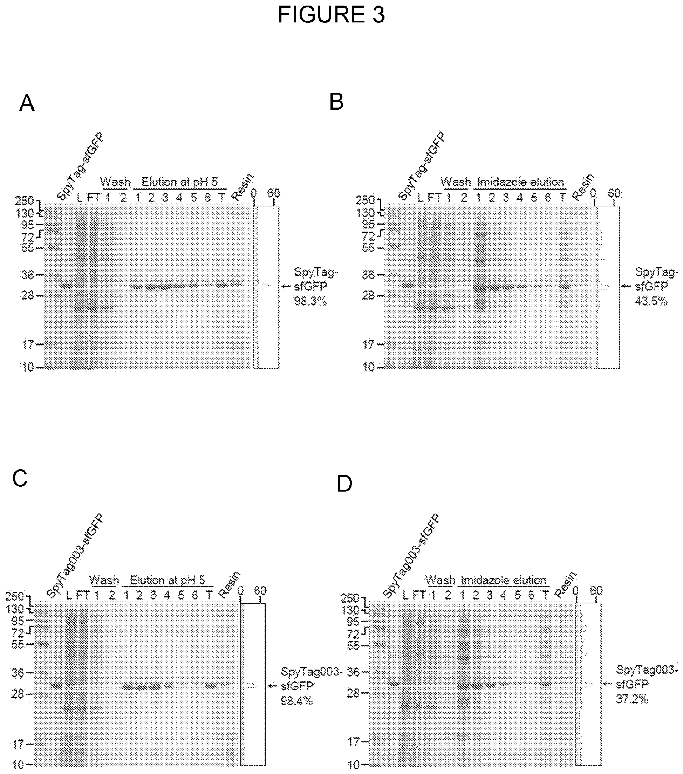

Figure 3: SpySwitch pH-dependent purification from bacterial expression. Figure 3A depicts purification of SpyTag-sfGFP by SpySwitch (SEQ ID NO: 16) after doping into *E. coli* lysate, eluting with pH 5.0 at 4 °C. Figure 3B depicts purification of SpyTag-sfGFP by Ni-NTA after doping into *E. coli* lysate. Figure 3C depicts purification of SpyTag003-sfGFP by SpySwitch after doping into *E. coli* lysate, eluting with pH 5.0 at 4 °C. Figure 3D depicts purification of SpyTag003-sfGFP by Ni-NTA after doping into *E. coli* lysate. Purification of sfGFP bearing a His₆-tag and SpyTag was tested after doping into *E. coli* lysate, assessed by reducing SDS-PAGE/Coomassie. L, doped lysate; FT, flow-through; T, total pooled elution fractions; Resin, protein left on resin following elution. On the right of each gel is the trace from densitometry of lane T and the percent purity.

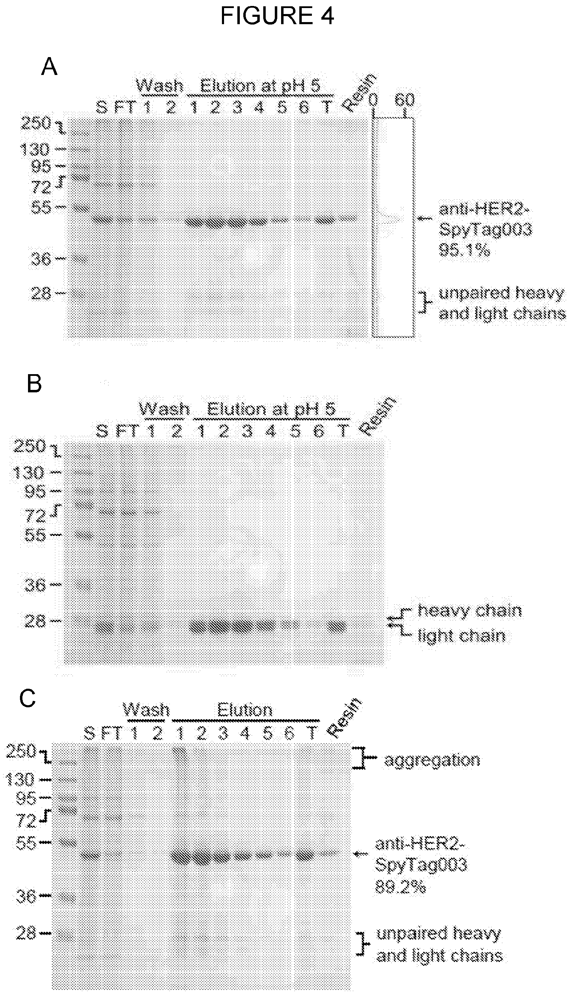

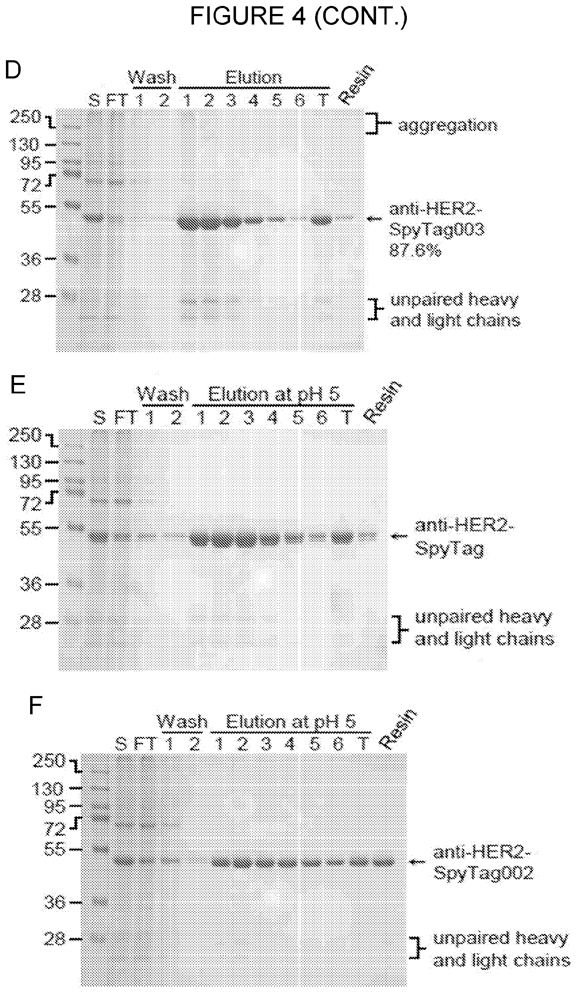

Figure 4: SpySwitch pH-dependent purification from mammalian expression. Figure 4A depicts purification of anti-HER2 Fab antibody fragment bearing a His₆-tag and SpyTag003 expressed in Expi293 cells using SpySwitch, with elution by pH 5.0 at 4 °C. Purity was assessed by non-reducing SDS-PAGE stained with Coomassie. The putative unpaired heavy and light chains of the anti-HER2 Fab are marked, based on close mobility to the reduced anti-HER2 Fab heavy and light chain in Figure 4B and these bands are absent in any other purifications from mammalian culture supernatant. Figure 4B depicts reducing SDS-PAGE/Coomassie of anti-HER2 Fab bearing SpyTag003 purified by SpySwitch, as shown in Figure 4A. Figures 4C and 4D depict purification of anti-HER2 bearing a His₆-tag and SpyTag003 expressed in Expi293 cells using SpyDock with elution in 2.5 M imidazole in Tris phosphate (TP) buffer pH 7.0 or Ni-NTA with elution in 200 mM imidazole in Ni-NTA binding buffer, respectively. Purity was assessed by non-reducing SDS-PAGE/Coomassie. S, Expi supernatant; FT, flow-through; T, total pooled elution fractions; Resin, protein left on resin following elution. Figures 4E and 4F depict purification of anti-HER2 Fab bearing a His₆-tag and SpyTag (Figure 4E) or SpyTag002 (Figure 4F) expressed in Expi293 cells using SpySwitch, with elution by pH 5.0 at 4 °C. Purity was assessed by non-reducing SDS-PAGE stained with Coomassie. S, Expi supernatant; FT, flow-through; T, total pooled elution fractions; Resin, protein left on resin following elution.

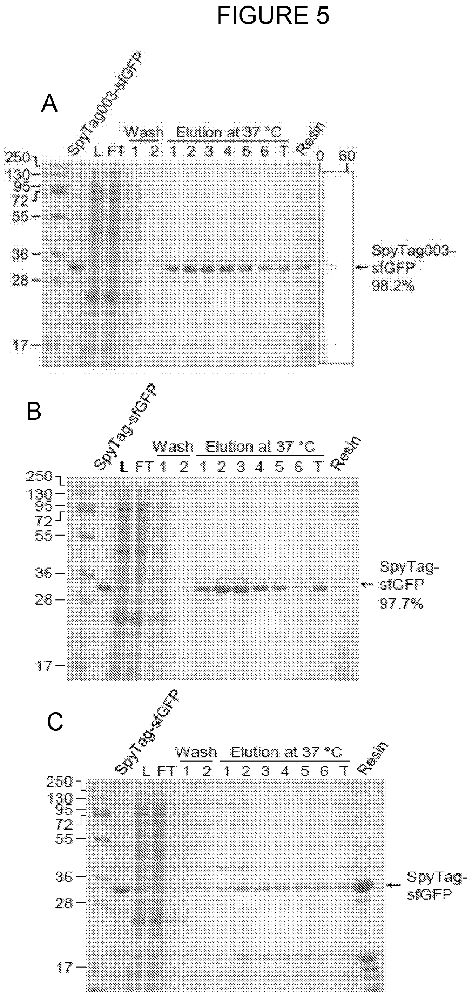

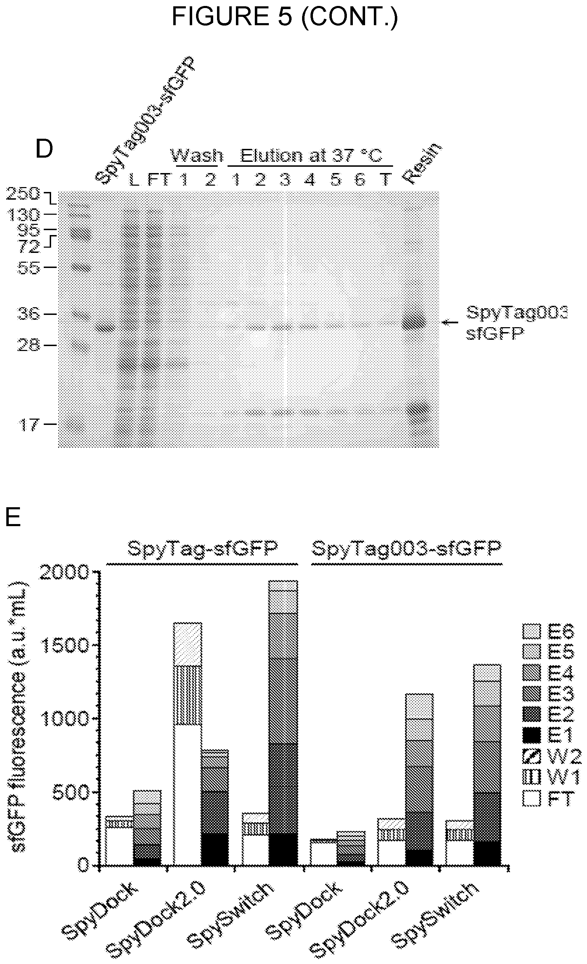

Figure 5: SpySwitch temperature-dependent purification from bacterial expression. Figure 5A depicts reducing SDS-PAGE performed for SpySwitch temperature elution of SpyTag003-sfGFP from bacterial lysate, with capture at 4 °C and elution at 37 °C. L, doped lysate; FT, flow-through; T, total pooled elution fractions; Resin, protein left on resin following elution. Figure 5B depicts reducing SDS-PAGE performed for SpySwitch temperature elution of SpyTag-sfGFP from bacterial lysate, with capture at 4 °C and elution at 37 °C. L, doped lysate; FT, flow-through; T, total pooled elution fractions; Resin, protein left on resin following elution. Figure 5C depicts SpyDock temperature elution of SpyTag-sfGFP as in Figure 5B. Figure 5D depicts SpyDock temperature elution of SpyTag003-sfGFP as in Figure 5A. Figure 5E shows that SpySwitch and SpyDock2.0 but not SpyDock allow temperature elution. SpyTag-sfGFP or SpyTag003-sfGFP was captured by SpySwitch, SpyDock2.0 or SpyDock at 4 °C. Fluorescence was determined from flow-through (FT), wash (W), or 37 °C elution (E) fractions. a.u.: arbitrary units.

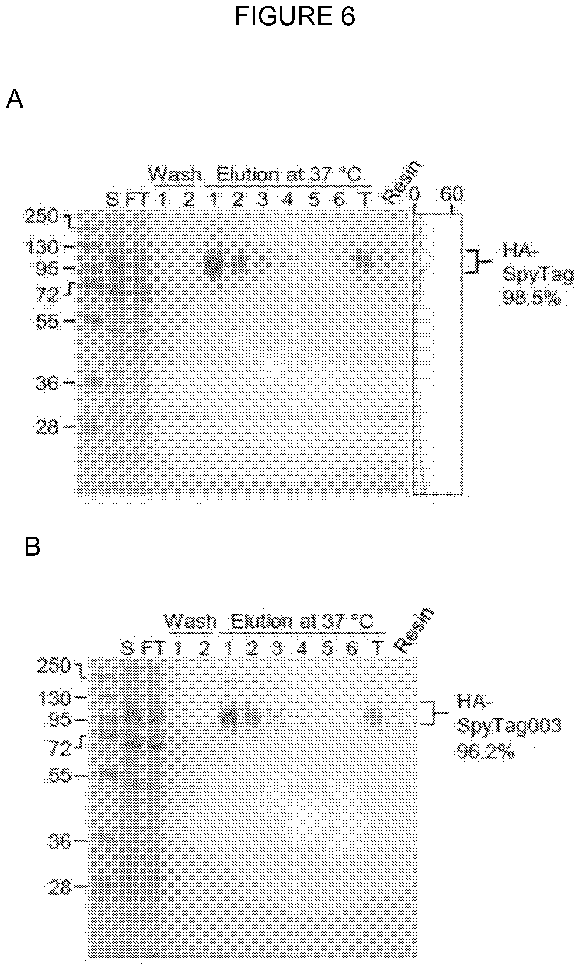

Figure 6: SpySwitch temperature-dependent purification from mammalian expression. Figure 6A depicts SpySwitch temperature elution of haemagglutinin (HA)-SpyTag from mammalian Expi293 expression, by reducing SDS-PAGE/Coomassie. S, supernatant; FT, flow-through; T, total pooled elution fractions; Resin, protein left on resin following elution. Figure 6B depicts reducing SDS-PAGE performed for SpySwitch temperature elution of HA-SpyTag003 from mammalian Expi293 expression, with capture at 4 °C and elution at 37 °C. S, supernatant; FT, flow-through; T, total pooled elution fractions; Resin, protein left on resin following elution.

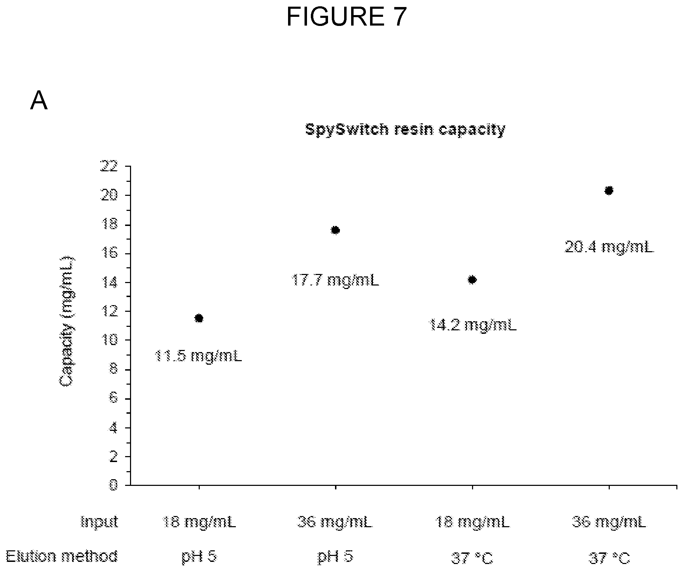

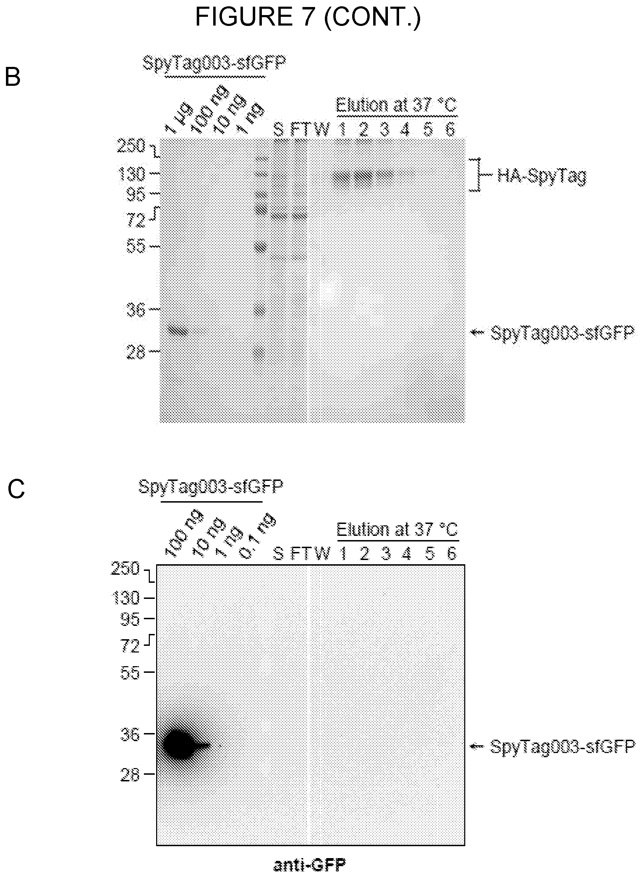

Figure 7: SpySwitch resin capacity and regeneration. Figure 7A depicts SpySwitch resin capacity, which was determined by doping SpyTag-sfGFP into bacterial lysate at 18 mg/mL or 36 mg/mL and purifying by pH switch at pH 5.0 or temperature switch at 37 °C. Protein concentration was measured by A₂₈₀ after elution and the yield was calculated. In Figure 7B, SpyTag003-sfGFP was bound to SpySwitch resin and regeneration was performed by sequential washes with 0.1 M glycine-HCl pH 2.0, 50 mM Tris-HCl pH 7.5 + 8 M urea, and 0.1 M NaOH. Regenerated SpySwitch resin was used for purification of HA-SpyTag from mammalian Expi293 expression by temperature elution at 37 °C. S, Expi supernatant; FT, flow-through; W, wash. Figure 7B shows the results of reducing SDS-PAGE/Coomassie. Figure 7C shows the results of reducing Western blot stained with anti-GFP. SpyTag003-sfGFP was loaded in varying amounts as a standard to allow for calibration.

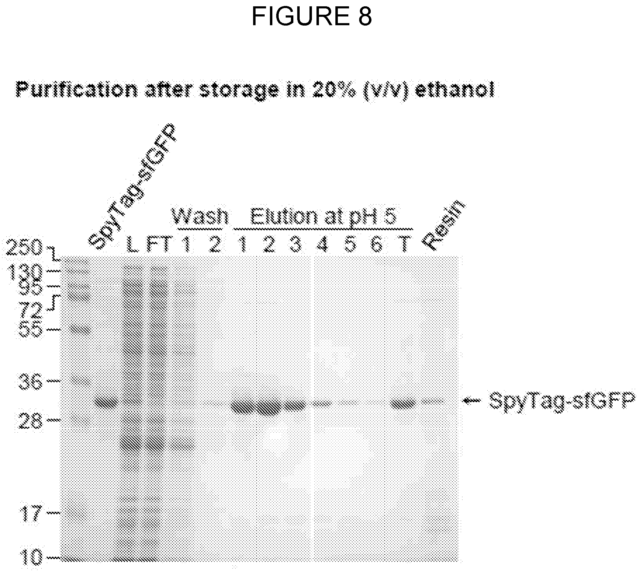

Figure 8: SpySwitch resin is stable to storage. SpySwitch resin was stored in 20% (v/v) ethanol in TP buffer pH 7.0 at 4 °C for 7 weeks, then used for purification of SpyTag-sfGFP doped into *E. coli* lysate. Reducing SDS-PAGE/Coomassie results are shown. L, doped lysate; FT, flow-through; T, total pooled elution fractions; Resin, protein left on resin following elution.

### EXAMPLES

### Example 1 - Mutation of SpyCatcher003 to generate a pH-switchable

### SpyTag-binder

Histidine residues were introduced to an unreactive SpyCatcher003 (S49C, E77A) polypeptide at the binding interface of SpyTag/SpyCatcher. Based on the crystal structure, 16 residues were identified on SpyCatcher adjacent to the SpyTag binding site. These residues were individually mutated to histidine. These single histidine mutants of SpyCatcher003 S49C E77A were coupled to resin and assayed for binding to SpyTag003-superfolder green fluorescent protein (sfGFP) at pH 8.0, with sequential elution at pH 6.0, pH 5.0 and pH 4.0. The original SpyDock, which shows little pH-sensitivity, was used for comparison. Elution fractions were neutralised, and the fluorescence of sfGFP was measured. As shown in Figure 2A, most of the mutants did not have a substantial effect on pH elutability. Of the six mutations that showed a promising effect, five (S30H, Y84H, G83H, E34H and E85H) were selected to make ten double histidine mutants.

Figure 2B demonstrates that of the ten double mutants created, mutants S30H + E85H, S30H + G83H and S30H + Y84H showed the most promise, with increased elutability at pH 5.0, but without the compromised binding at pH 8.0 seen for S30H + E34H. These encouraging double mutants were combined into three triple mutants; S30H + Y84H + E85H showed best pH elutability at pH 5.0 and 6.0 (Figure 2C). Therefore, the three mutations S30H, Y84H and E85H were added into SpyCatcher003 S49C E77A, to generate a variant which was termed SpyDock2.0 (SEQ ID NO: 10). This variant allows capture of SpyTag003-fused proteins at neutral pH before eluting under weakly acidic conditions (Figures 1A and 2).

### Example 2 - Enhancing pH-responsiveness by phage display selection

Individually mutating and combining residues has the limitation that cooperativity between different residues is difficult to assess, without expressing and testing an extensive library of variants. To optimise a SpyDock variant polypeptide that shows strong binding at pH 8.0 but high elutability at pH 5.0 and 6.0, a phage display library of SpyDock2.0 was created. A C49S back-mutation was included to avoid disulfide-mediated dimer formation on phage. Binding of biotinylated SpyTag003-MBP bait was performed at pH 8.0. For initial rounds, elution was performed at pH 5.0. In later rounds the elution pH was increased gradually, up to pH 7.0. Display on phage was optimised after inducing with different concentrations of arabinose and validated by Western blot using an antibody against the HA tag on the N-terminus of SpyDock2.0, as well as with polyclonal anti-SpyCatcher serum.

Having optimised display, error-prone PCR was performed using Mutazyme II to generate phage library 1 from SpyDock2.0 C49S, with induction in the presence of 0.2% arabinose. After four rounds of selection using library 1, three mutations R32H, S59T and A111P were seen repeatedly (Table 1). Whilst not wishing to be bound by theory, it is thought that the S59T mutation, possibly in combination with A111P, had a significant effect on stabilising the interaction between SpyTag and the mutated SpyDock polypeptide.

**Table 1**

| | | | | | | | | |
|---|---|---|---|---|---|---|---|---|
| Library 1 | Mutations relative to SpyDock2.0 | | | | | | | |
| Round 4, clone 1 | G12R | | R32H | | | H84Y | | A111P |
| Round 4, clone 4 | | | R32H | S55C | S59T | | | |
| Round 4, clone 6 | M17L | T18A | R32H | S55R | S59T | | | |
| Round 4, clone 9 | | S23R | R32H | | S59T | H85N | | A111P |
| Round 4, clone 10 | | | R32H | T43A | | | E96G | A111P |
| Selected mutations | | | R32H | | S59T | | | A111P |

### Example 3 - Further increasing binding stability by phage display selection

A second phage display library was then constructed based on SpyDock2.0 R32H S59T A111P. Library 2 used either the original codons or a template using very different codons, to allow for a greater number of amino acids to be accessed through mutation of a single base in the error-prone PCR. Several mutations from the second library were screened, but only V94I was included to further increase binding stability (Table 2). Of the mutations added from phage display selections, R32H and A111P are in direct proximity to SpyTag, while S59T and V94I are distant from the SpyTag binding site.

**Table 2**

| | | | | |
|---|---|---|---|---|
| Library 2 | Mutations relative to SpyDock2.0 R32H S59T A111P | | | |
| Round 2, original codons | | V94I | | T101A |
| Round 2, alternative codons | S50R | V94I | D97V | |
| Round 3, original codons | | V94I | | |
| Selected mutations | | V94I | | |

### Example 4 - Further improving pH elutability

The effect of a histidine mutation may be affected by the residues surrounding it. Therefore, several mutations from the initial histidine mutagenesis screen were retested in the context of the best phage-derived sequence (SpyDock2.0 R32H S59T V94I A111P). K28H was found to improve pH elutability, especially for SpyTag003, and was added into the final SpySwitch sequence (SEQ ID NO: 6). The N-terminal region was further modified by removing the tobacco etch virus (TEV) protease cleavage site and preceding amino acids, leaving a His₆-tag and (GSG)₂ linker before the SpySwitch sequence (SEQ ID NO: 16).

### Example 5 - SpySwitch pH-dependent purification from bacterial expression

pH-dependent purification was initially tested from *Escherichia coli* lysate. Affinity purification of highly expressed proteins is much easier than for low abundance proteins. Therefore, a challenging situation was set up by doping 0.09 mg of N-terminally tagged SpyTag-sfGFP or SpyTag003-sfGFP into 0.25 g of *E*. *coli* wet cell weight, mimicking a poorly expressed protein. This approach also allowed direct comparison between the two tags, since SpyTag-sfGFP and SpyTag003-sfGFP might not show identical levels of expression. Both proteins also contained a His₆-tag at the C-terminus to allow comparison with the most widely used method of nickel-nitrilotriacetic acid (Ni-NTA) purification.

For SpySwitch, elution was performed with pH 5.0 buffer at 4 °C. It was observed that both SpyTag-sfGFP and SpyTag003-sfGFP purification occurred at greater than 98% purity (Figure 3A and 3C). SpySwitch gave considerably better purity than standard Ni-NTA resin, which only gave less than 50% purity using this low level of target protein (Figure 3B and 3D).

Overall, it has been shown that SpyTagged proteins can be purified efficiently by SpySwitch using mild pH elution from bacterial expression systems at high purity.

### Example 6 - SpySwitch pH-dependent purification from eukaryotic expression

To test SpySwitch pH-dependent purification from eukaryotic expression, an anti-HER2 Fab antibody fragment was transiently expressed in the human embryonic kidney-based Expi293 cell-line (Figure 4A and 4B). The construct was tagged with a C-terminal SpyTag003 on the heavy chain, as well as a His₆-tag. SpySwitch purification was efficient, with higher purity observed for SpySwitch (95.1%) than either SpyDock (89.2%, using 2.5 M imidazole elution, Figure 4C) or Ni-NTA (87.6%, using 200 mM imidazole elution, Figure 4D). These different purification methods also gave a product just below 28 kDa, which is likely to be Fab that has not formed a disulfide, based on reducing SDS-PAGE (Figure 4B). SpySwitch purification of anti-HER2 Fab with a C-terminal SpyTag (Figure 4E) and of an anti-HER2 Fab-SpyTag002 (Figure 4F) was also effective.

Overall, it has been shown that SpyTagged proteins can be purified efficiently by SpySwitch using mild pH elution from mammalian expression systems at high purity.

### Example 7 - Biophysical characterisation of SpySwitch and its interactions

Biophysical validation of the SpySwitch construct was performed to get insight into this successful purification behaviour. Electrospray ionisation mass spectrometry (ESI-MS) was used to confirm the molecular identity of SpySwitch followed by isothermal titration calorimetry (ITC) to investigate the binding affinity of SpySwitch. The interaction between monomeric SpySwitch and SpyTag-MBP as well as SpyTag003-MBP was tested at pH 7.5 and 5 °C. Both SpyTag-MBP and SpyTag003-MBP bound with 1:1 stoichiometry. SpyTag-MBP (K_{d} = 1.1 µM) bound SpySwitch slightly tighter than SpyTag003-MBP (K_{d} = 1.5 µM), suggesting that by optimising elutability at acidic pH, the binding affinity at neutral pH had also been lowered. No binding between SpySwitch and either SpyTag-MBP or SpyTag003-MBP was observed by ITC at pH 5.0 and 5 °C, suggesting that any interaction present had negligible ΔH or was weaker than the detection limit of ITC.

### Example 8 - SpySwitch temperature-dependence

Important viral antigens can be activated to switch conformation by endosomal pH. Therefore, for these proteins pH-dependent elution would not be suitable. Temperature-dependent elution using SpySwitch was investigated to provide an alternative method. Differential scanning calorimetry (DSC) was run on SpySwitch at different pH values. At pH 8.0, SpySwitch had a melting temperature of 33.4 °C, suggesting that SpySwitch might release its interaction with SpyTag- or SpyTag003-fusions upon heating up to 37 °C. The relationship between melting temperature and pH was also determined. Strikingly, SpySwitch's melting temperature decreased by 6-10 °C per pH unit, with the highest melting temperature (Tm) at pH 5.0 (Tm = 57.4 °C). To investigate if this temperature-dependence was an intrinsic feature of the scaffold, the unfolding of SpyDock was also tested. SpyDock's melting temperature was well above 37 °C at all pH values and showed less pH-sensitivity than SpySwitch.

### Example 9 - SpySwitch temperature-dependent purification at neutral pH

It was tested whether the temperature-dependence in SpySwitch could be utilised for purification close to neutral pH (Figure 1B). As described above, low levels of SpyTag003-sfGFP were doped into *E. coli* lysate, capturing in pH 7.5 buffer at 4 °C and eluting at pH 8.0 at 37 °C (Figure 5A). Results show that the SpyTag003 fusion was purified from the lysate at 98.2% purity (Figure 5A). The SpyTag fusion was similarly purified by temperature elution from *E. coli* lysate with 97.7% purity (Figure 5B). In comparison, temperature elution from SpyDock using the same conditions yielded only a small amount of elution, consistent with the melting temperature of SpyDock (Figures 5C and 5D).

SpySwitch (SEQ ID NO: 6) was compared against SpyDock2.0 (SEQ ID NO: 10) or SpyDock (SEQ ID NO: 15) for temperature elution of SpyTag-sfGFP and SpyTag003-sfGFP (Figure 5E), demonstrating that there was greater elution from SpySwitch. For SpyTag003-sfGFP, temperature elution worked well for SpyDock2.0 but there was little elution from SpyDock. However, binding of SpyTag-sfGFP to SpyDock2.0 was severely compromised (Figure 5E). Very little temperature elution of SpyTag-sfGFP and SpyTag003-sfGFP was seen from SpyDock (Figure 5E).

### Example 10 - SpySwitch temperature-dependent purification of a pH-sensitive vaccine antigen

To test purification of a pH-sensitive vaccine antigen, tagged trimeric influenza haemagglutinin (HA) was expressed in mammalian cells and purified from the supernatant, utilising temperature-switch elution at 37 °C. HA-SpyTag was isolated at 98.5% purity by temperature elution (Figure 6A), while HA-SpyTag003 was isolated to 96% purity (Figure 6B).

### Example 11 - SpySwitch resin capacity and regeneration

SpySwitch resin capacity was 17.7 mg protein per mL of packed resin when eluted from SpySwitch using the pH 5.0 elution. Recovery increased to 20.4 mg when using 37 °C elution (Figure 7A). It was also shown that the resin could be successfully regenerated. SpySwitch resin was used to purify SpyTag003-sfGFP and then regenerated with washes of glycine-HCl pH 2.0, then 8 M urea, before a final wash in 0.1 M sodium hydroxide. The regenerated resin was applied for purification of HA-SpyTag. HA-SpyTag was successfully purified and residual SpyTag003-sfGFP was not detected, based on Coomassie stain or Western blotting against sfGFP (Figures 7B and 7C).

### Example 12 - Storage of SpySwitch

SpySwitch was stored in Tris-phosphate (TP) buffer with 20% (v/v) ethanol at 4 °C, to stop microbial growth. SpySwitch maintained good performance over time, based on purification of SpyTag-sfGFP following storage for 7 weeks (Figure 8).

### Conclusion

The original SpyDock polypeptide has numerous limitations on its utility in affinity purification. In particular SpyDock required stringent elution conditions that are disruptive to the folding and assembly of many proteins.

As described above, the development of SpySwitch resulted from initially testing a large series of histidine-scanning mutants and selection of suitable mutations to give SpyDock 2.0. It was found that SpyDock2.0 does not work equally well with all SpyTag peptides, e.g. SpyDock2.0 shows excellent capture of SpyTag003-fusions and mediocre capture of SpyTag-fusions, followed by easy elution of SpyTag-fusions and only partial elution of SpyTag003-fusions. Thus, development of SpySwitch required further engineering of SpyDock2.0 to obtain a system that works well for both SpyTag and SpyTag003, enabling application of a single resin. However, this posed a major challenge because SpyTag003 was engineered to have much stronger binding to its Catcher than SpyTag. Therefore, further screening and selection of the multiple arising mutants was required, employing an innovative phage screening system with sequential tuning of elution conditions. The final polypeptide, SpySwitch, represents a successful compromise construct that provides good purification for both SpyTag and SpyTag003, showing similar ~1 µM affinity for each tag. In contrast, SpyCatcher003 interacts with the non-reactive SpyTag003 D117A fused to MBP with ~10 nM K_{d}. These data suggest that, by optimising SpyTag003 elutability at acidic pH, we also lowered SpyTag003's binding affinity for SpySwitch at neutral pH.

### Methods

### Resin coupling

SpySwitch (and variants thereof) were coupled to SulfoLink Coupling Resin (Thermo Fisher) according to the manufacturer's instructions with some modifications. Briefly, SpySwitch was concentrated to at least 20 mg/mL using a Vivaspin 20 5 kDa molecular weight cut-off (MWCO) centrifugal filter at 4 °C and reduced with 1 mM TCEP in coupling buffer (50 mM Tris-HCl + 5 mM EDTA, adjusted to pH 8.5 at 25 °C) for at least 30 min at 25 °C. Resin coupling was performed at 25 °C throughout, with incubations protected from light. 20 mg reduced SpySwitch was added per 1 mL packed SulfoLink Coupling Resin in coupling buffer containing 1 mM TCEP and incubation was performed first for 30 min on an end-to-end rotator, then for a further 30 min without rotation. After washing with coupling buffer, the column was blocked with 50 mM L-cysteine in coupling buffer by incubating for 15 min on an end-to-end rotator, then for a further 30 min without rotation. Washes with 1 M NaCl, then additionally TP buffer (25 mM orthophosphoric acid adjusted to pH 7.0 with Tris base) were performed to remove non-covalently-bound SpySwitch. SpySwitch resin was stored in 20% (v/v) ethanol in TP buffer.

### pH elution assay by fluorescence

To test binding and elution properties of SpyDock variants, 30 µL packed SpyCatcher003 S49C E77A histidine mutant resins were incubated with 50 µg SpyTag-sfGFP or SpyTag003-sfGFP in a total volume of 300 µL. For the histidine mutant screen performed in Figure 2, 50 mM 4-(2-hydroxyethyl)-1-piperazineethanesulfonic acid (HEPES) pH 8.0 + 150 mM NaCl was used for binding and washing. Samples were incubated for 45 min at 4 °C with end-over-end rotation. The resin was collected by centrifugation at 1,000 g for 5 min at 4 °C, then transferred to wells of a pre-wetted AcroPrep Advance 0.45 µm Supor membrane 96-well plate (Pall Corporation). The flow-through was collected by centrifugation at 500 g and 4 °C for 30 s. The resin was washed four times with 250 µL wash buffer by centrifugation at 500 g and 4 °C for 30 s. For stepwise elution, resins were sequentially incubated in 2× 50 µL 50 mM 2-(N-morpholino)ethanesulfonic acid (MES) pH 6.0 + 150 mM NaCl; then 50 mM acetic acid/sodium acetate pH 5.0 + 150 mM NaCl; then 50 mM acetic acid/sodium acetate pH 4.0 + 150 mM NaCl for 10 min 4 °C. Elution fractions were neutralised by addition of 1 M Tris-HCl pH 8.0 to the collector plates (23 µL for pH 6.0, 10 µL for pH 5.0, 20 µL for pH 4.0) and volumes adjusted to 73 µL with the respective pH buffer that had been pre-neutralised by addition of 1 M Tris-HCl pH 8.0. 20 µL of each sample was transferred to black, flat-bottom half area 96-well plates (Corning) and sfGFP left to incubate at 4 °C for 30 min. Fluorescence was measured using a CLARIOstar plate reader (BMG Labtech) (λex = 482 ± 16 nm, λem = 530 ± 40 nm) at 30 °C. Experiments were performed in triplicate and the mean ± 1 standard deviation (s.d.) was calculated.

### Protein purification by Ni-NTA

Purifications were performed at 4 °C. Bacterial cell pellets were thawed and resuspended in Ni-NTA buffer (50 mM Tris-HCl pH 7.8 + 300 mM NaCl) supplemented with cOmplete Mini EDTA-free Protease Inhibitor Cocktail (Roche) and 1 mM phenylmethylsulfonylfluoride (PMSF, Thermo Fisher). For the histidine mutants, incubation with 100 µg/mL lysozyme and 2 U/mL benzonase (both Sigma-Aldrich) was performed for 30 min, rotating at 25 °C. Cells were lysed by sonication on ice at 50% duty cycle for 4 × 1 min with 1 min rest between runs. SpySwitch (and variants thereof), SpyDock2.0 and SpyDock lysates were supplemented with 10 mM 2-mercaptoethanol. Cell lysates were clarified by centrifugation at 30,000 g for 30 min at 4 °C, then incubated with Ni-NTA agarose (Qiagen) for 1 h at 4 °C rolling. Resin was pelleted by centrifugation, then washed with 20 column volumes (CV) of Ni-NTA buffer and Ni-NTA wash buffer (10 mM imidazole in Ni-NTA buffer), supplemented with 10 mM 2-mercaptoethanol for SpySwitch and SpyDock. The resin was transferred to Econo-Pac Chromatography Columns (Bio-Rad) in Ni-NTA wash buffer and washed with a further 20 CV of Ni-NTA wash buffer by gravity flow. Elution was performed with Ni-NTA elution buffer (200 mM imidazole in Ni-NTA buffer) by incubating the resin in at least 4× 1 CV for 5 min. Protein concentrations were determined by A₂₈₀ measurement on a NanoDrop One (Thermo Fisher), using extinction coefficients predicted by ExPASy ProtParam.

SpySwitch, SpyDock2.0 and SpyDock were further purified by size-exclusion chromatography using a HiLoad 16/600 Superdex 75 pg column at 1 mL/min flow rate in 50 mM Tris-HCl pH 8.5, 5 mM ethylenediamine tetraacetic acid (EDTA), 1 mM TCEP. Where needed, dialysis was performed into appropriate buffers using 3.5 kDa MWCO dialysis tubing (Spectrum Labs). Proteins were concentrated using Vivaspin 6 or 20 centrifugal devices with 5 kDa or 10 kDa MWCO (Cytiva). Typical protein yields per L culture were 20-30 mg for SpySwitch, SpyDock2.0 and SpyDock, 11 mg for SpyTag-sfGFP, and 6 mg for SpyTag003-sfGFP.

### Purification by SpySwitch

For bacterial lysate doping, untransfected *E. coli* BL21 (DE3) RIPL were grown in LB medium to A₆₀₀ 0.5 and incubated with 420 µM IPTG at 18 °C and 200 rpm for 16 h. Per gram of wet cell weight, 2 mL SpySwitch wash buffer (50 mM Tris-HCl pH 7.5 + 300 mM NaCl) supplemented with cOmplete Mini EDTA-free Protease Inhibitor Cocktail and 1 mM PMSF was added and cells were lysed by sonication on ice at 50% duty cycle for 4 × 1 min, with 1 min rest after each run. The lysate was clarified by centrifugation at 30,000 g for 30 min at 4 °C and adjusted to pH 7.5 with 1 M Tris-HCl pH 8.0. Lysate was used immediately or stored at -80 °C. For doping, 90 µg SpyTag-sfGFP or SpyTag003-sfGFP in a small volume of 25 mM Tris-HCl pH 8.0 +150 mM NaCl was added to a final volume of 500 µL RIPL lysate. On the gel analysis, this doped lysate was denoted as L. For purification from mammalian culture supernatant, 10% 10× SpySwitch buffer was added, making a final 50 mM Tris-HCl pH 7.5 + 300 mM NaCl. This adjusted supernatant is denoted as S on the gel analysis. SpySwitch resin was pre-equilibrated with 2× 10 CV SpySwitch buffer, then incubated with doped lysate or supernatant for 1 h at 4 °C on an end-over-end rotator. Small-scale purifications were performed in Micro Bio-Spin Chromatography Columns, while large-scale purifications were performed in Econo-Pac Chromatography Columns (both Bio-Rad). The column was drained with the flow-through (FT) collected and washed with 4× 10 CV SpySwitch buffer (collected as washes 1 and 2). Elution was performed in either of two ways.

Utilising the pH switch, bound protein was eluted with 6× 1.5 CV of 50 mM acetic acid/sodium acetate pH 5.0 + 150 mM NaCl at 4 °C, incubating each fraction for 5 min at 4 °C, into 0.3 CV 1 M Tris-HCl pH 8.0. Using the temperature switch, bound protein was eluted with 6× 1.5 CV of 50 mM HEPES pH 8.0 + 150 mM NaCl at 37 °C, incubating each fraction for 5 min at 37 °C.

When comparing SpySwitch against Ni-NTA and SpyDock purification, the same protocol was followed, however buffers were substituted. For Ni-NTA, washes were performed with 10 mM imidazole in 1× Ni-NTA buffer (or 1× Ni-NTA buffer for anti-HER2 Fab) and elution with 200 mM imidazole in 1× Ni-NTA buffer. For SpyDock, washes were performed with TP buffer pH 7.0 and elution with 2.5 M imidazole in TP buffer at pH 7.0. To allow for direct comparison, elution fractions from temperature, Ni-NTA or SpyDock elution were adjusted to the same volume as the neutralised pH elution fractions by addition of 0.3 CV of respective elution buffers. For total elution as shown on SDS-PAGE, equal amounts of each elution fraction were mixed. To assess remaining protein on resin after elution, the resin was resuspended 1:1 in PBS pH 7.4 and one tenth was analysed by SDS-PAGE.

### Regeneration and storage of SpySwitch resin

50 µL SpySwitch resin was incubated with 900 µg SpyTag003-sfGFP in bacterial lysate for 1 h at 4 °C. Regeneration was performed at 25 °C directly after binding. The resin was equilibrated with 2× 10 CV SpySwitch wash buffer and reequilibration was performed between the following regeneration steps: incubated with 3× 10 CV of 0.1 M glycine-HCl pH 2.0 for 5 min each, re-equilibrated, incubated with 3× 10 CV of 50 mM Tris, 8 M urea, pH 7.5 for 5 min each, re-equilibrated, incubated with 3× 10 CV of 0.1 M NaOH for 1 min each. The resin was re-equilibrated in SpySwitch wash buffer and incubated with 500 µL supplemented HA-SpyTag Expi293 supernatant at 4 °C. Washing and temperature elution were performed as described above. Elution fractions were assessed by Western blot for the presence of residual sfGFP using known amounts of sfGFP (100 ng, 10 ng, 1 ng and 0.1 ng) for comparison.

### SDS-PAGE and quantification

SDS-PAGE was performed using an XCell SureLock system (Thermo Fisher) with 12% or 14% polyacrylamide gels. Samples were prepared in a final concentration of 39 mM Tris-HCl pH 6.8, 4% (v/v) glycerol, 20 µM bromophenol blue, 39 mM SDS, supplemented with 10 mM dithiothreitol (DTT) for reduced samples. Samples were heated at 95 °C for 5 min in a Bio-Rad C1000 thermal cycler. Gels were run in 24 mM Tris base, 192 mM glycine, 3.5 mM SDS at 200 V and stained with Coomassie Brilliant Blue G-250. After destaining in MilliQ water, gels were imaged using a ChemiDoc XRS+ imager, and analysed with ImageLab version 3.0 (both Bio-Rad). For quantification of protein purity in ImageLab, high sensitivity band detection was performed. Additional bands were added manually where the automatic detection had failed to detect faint bands. 1 mm background subtraction was applied. The percentage purity is quantified as (band intensity of protein of interest)/(sum of the band intensities from all bands in lane)×100.

## Claims

1. A polypeptide comprising:
(i) an amino acid sequence as set forth in SEQ ID NO: 1, wherein X at position 77 is selected from alanine, glycine, serine, asparagine, or threonine;
(ii) a portion of (i) comprising an amino acid sequence as set forth in SEQ ID NO: 2, wherein X at position 56 is selected from alanine, glycine, serine, asparagine or threonine;
(iii) an amino acid sequence with at least 80% sequence identity to a sequence as set forth in SEQ ID NO: 1, wherein the polypeptide comprises alanine, glycine, serine, asparagine or threonine at a position equivalent to position 77 of SEQ ID NO: 1, and wherein the polypeptide comprises histidine at positions equivalent to positions 30, 84 and 85 of SEQ ID NO: 1; or
(iv) a portion of (iii) comprising an amino acid sequence with at least 80% sequence identity to a sequence as set forth in SEQ ID NO: 2, wherein the polypeptide comprises alanine, glycine, serine, asparagine or threonine at a position equivalent to position 56 of SEQ ID NO: 2, and wherein the polypeptide comprises histidine at positions equivalent to positions 9, 63 and 64 of SEQ ID NO: 2,
wherein the polypeptide binds selectively and reversibly to a peptide comprising an amino acid sequence as set forth in SEQ ID NO: 3, 4 or 5.

2. The polypeptide of claim 1, wherein X at a position equivalent to position 77 of SEQ ID NO: 1 or position 56 of SEQ ID NO: 2:
a) is selected from alanine, glycine or serine; or
b) is alanine.

3. The polypeptide of claim 1 or 2, wherein the polypeptide comprises an amino acid sequence with at least 80% sequence identity to a sequence as set forth in SEQ ID NO: 1, and wherein the amino acid sequence comprises:
a) histidine at one or both positions equivalent to positions 28 and 32 of SEQ ID NO: 1;
b) one or both of: (i) a glutamic acid at a position equivalent to position 91 of SEQ ID NO: 1; and (ii) an aspartic acid at a position equivalent to position 103 of SEQ ID NO: 1; and/or
c) one or more of: (i) a threonine at a position equivalent to position 59 of SEQ ID NO: 1; (ii) an isoleucine at a position equivalent to position 94 or SEQ ID NO: 1; and (iii) a proline at a position equivalent to position 111 of SEQ ID NO: 1.

4. The polypeptide of any one of claims 1 to 3, wherein the polypeptide comprises an amino acid sequence with at least 80% sequence identity to a sequence as set forth in SEQ ID NO: 1 and wherein the amino acid sequence comprises:
A) all of the following:
i) histidine at positions equivalent to positions 28, 30, 32, 84 and 85 of SEQ ID NO: 1;
ii) threonine at a position equivalent to position 59 of SEQ ID NO: 1;
iii) glutamic acid at a position equivalent to position 91 of SEQ ID NO: 1;
iv) isoleucine at a position equivalent to position 94 of SEQ ID NO: 1;
v) aspartic acid at a position equivalent to position 103 of SEQ ID NO: 1; and
vi) proline at a position equivalent to position 111 of SEQ ID NO: 1;
B) one or more of the following:
i) lysine at a position equivalent to position 31 of SEQ ID NO: 1;
ii) proline at a position equivalent to position 89 of SEQ ID NO: 1;
iii) aspartic acid at a position equivalent to position 97 of SEQ ID NO: 1; and
iv) glutamic acid at a position equivalent to position 108 of SEQ ID NO: 1;
C) lysine at position 31, proline at position 89, aspartic acid at position 97, glutamic acid at position 108 and one or more of the following:
i) threonine at position 2;
ii) glycine at position 9;
iii) proline at position 13;
iv) threonine at position 19;
v) arginine at position 37;
vi) histidine at position 62;
vii) glutamic acid at position 105; and
viii) threonine at position 113,
wherein the specified amino acid residues are at positions equivalent to the positions in SEQ ID NO: 1; and/or
D)
i) threonine at position 2;
ii) glycine at position 9;
iii) proline at position 13;
iv) threonine at position 19;
v) lysine at position 31;
vi) arginine at position 37;
vii) histidine at position 62;
viii) proline at position 89;
ix) aspartic acid at position 97;
x) glutamic acid at position 105;
xi) glutamic acid at position 108; and
xii) threonine at position 113,
wherein the specified amino acid residues are at positions equivalent to the positions in SEQ ID NO: 1.

5. The polypeptide of any one of claims 1 to 4, wherein the polypeptide comprises an amino acid sequence with at least 80% sequence identity to a sequence as set forth in SEQ ID NO: 1 and wherein the amino acid sequence comprises cysteine at a position equivalent to position 49 in SEQ ID NO: 1 or position 28 in SEQ ID NO: 2.

6. The polypeptide of any one of claims 1 to 5, wherein the polypeptide is immobilised on a solid substrate, preferably wherein the polypeptide is immobilised on the solid substrate via a covalent bond, optionally wherein the polypeptide comprises an amino acid sequence with at least 80% sequence identity to a sequence as set forth in SEQ ID NO: 1 and wherein the amino acid sequence comprises cysteine at position 49 and the polypeptide is immobilised on a solid substrate via a covalent bond between said cysteine and the solid substrate, wherein the specified amino acid residue is at a position equivalent to the position in SEQ ID NO: 1.

7. A nucleic acid molecule comprising a nucleotide sequence which encodes a polypeptide as defined in any of claims 1 to 6.

8. A vector comprising the nucleic acid molecule of claim 7.

9. A cell comprising the nucleic acid molecule of claim 7 or vector of claim 8.

10. A process for purifying or isolating a molecule or component comprising a peptide having an amino acid sequence with at least 80% sequence identity to a sequence as set forth in one of SEQ ID NOs: 3-5, preferably wherein said peptide comprises an aspartic acid at a position equivalent to position 7 of SEQ ID NO: 3, position 8 of SEQ ID NO: 4 or position 10 of SEQ ID NO: 5, said process comprising:
a) providing a solid substrate on which a polypeptide of any one of claims 1 to 6 is immobilised;
b) providing a sample comprising said molecule or component;
c) contacting the solid substrate of a) with the sample of b) under conditions that enable said peptide to selectively bind to said polypeptide, thereby forming a non-covalent complex between said polypeptide immobilised on the solid substrate and molecule or component comprising said peptide;
d) washing the solid substrate with a wash solution; and
e) separating the molecule or component comprising the peptide from the polypeptide immobilised on the solid substrate.

11. The process of claim 10, wherein:
a) the step of contacting the solid substrate of a) with the sample of b) occurs under the conditions of:
i) a temperature of about 0-25 °C, preferably about 0-15 or about 0-10 °C; and
ii) a neutral pH, preferably a pH of about 6.5-8.5, preferably about 7.0-8.0 or about 7.3-7.7;
b) the step of washing the solid substrate with a wash solution comprises contacting said solid substrate with a pH neutral wash solution, optionally a buffered solution with a pH of about 6.5-8.5, preferably about 7.0-8.0 or about 7.3-7.7; and/or
c) the step of separating the molecule or component comprising the peptide comprises:
(i) contacting said complex with an acidic solution, optionally a buffered solution with a pH of about 3.0-6.0, preferably about 3.5-5.5; and/or
(ii) increasing the temperature of said complex to at least about 25 °C, preferably about 25-45 °C, 30-40 °C or 35-45 °C, optionally comprising contacting said complex with a solution having a temperature of at least about 25 °C, preferably about 25-45 °C, 30-40 °C or 35-45 °C; and/or
(iii) contacting said complex with a solution comprising a peptide comprising or consisting of an amino acid sequence with at least 80% sequence identity to an amino acid sequence as set forth in one of SEQ ID NOs: 3-5, preferably a peptide comprising or consisting of an amino acid sequence as set forth in one of SEQ ID NOs: 3-5.

12. Use of a polypeptide as defined in any one of claims 1 to 6 to purify or isolate a molecule or component comprising a peptide having an amino acid sequence with at least 80% sequence identity to a sequence as set forth in one of SEQ ID NOs: 3-5, preferably wherein said peptide comprises an aspartic acid at a position equivalent to position 7 of SEQ ID NO: 3, position 8 of SEQ ID NO: 4 or position 10 of SEQ ID NO: 5.

13. An apparatus suitable for use in the process of claims 10 or 11 or use of claim 12 comprising a solid substrate on which a polypeptide as defined in any one of claims 1 to 6 is immobilised.

14. A kit suitable for use in preparing a solid substrate on which a polypeptide as defined in any one of claims 1 to 6 is immobilised, comprising:
a) a polypeptide as defined in any one of claims 1 to 6; and
b) means for immobilising the polypeptide of a) on a solid substrate, optionally wherein the kit further comprising a solid substrate.

15. A resin and/or beads for use in protein purification methods on which a polypeptide as defined in any one of claims 1 to 6 is immobilised.

## Patentansprüche

1. Polypeptid, umfassend:
(i) eine Aminosäuresequenz gemäß SEQ ID NO: 1, wobei X an Position 77 aus Alanin, Glycin, Serin, Asparagin oder Threonin ausgewählt ist;
(ii) einen Teil von (i), der eine Aminosäuresequenz gemäß SEQ ID NO: 2 umfasst, wobei X an Position 56 aus Alanin, Glycin, Serin, Asparagin oder Threonin ausgewählt ist;
(iii) eine Aminosäuresequenz mit mindestens 80 % Sequenzidentität zu einer Sequenz gemäß SEQ ID NO: 1, wobei das Polypeptid Alanin, Glycin, Serin, Asparagin oder Threonin an einer Position umfasst, die der Position 77 der SEQ ID NO: 1 entspricht, und wobei das Polypeptid Histidin an Positionen umfasst, die den Positionen 30, 84 und 85 der SEQ ID NO: 1 entsprechen; oder
(iv) einen Teil von (iii), der eine Aminosäuresequenz mit mindestens 80 % Sequenzidentität zu einer Sequenz gemäß SEQ ID NO: 2 umfasst, wobei das Polypeptid Alanin, Glycin, Serin, Asparagin oder Threonin an einer Position umfasst, die der Position 56 der SEQ ID NO: 2 entspricht, und wobei das Polypeptid Histidin an Positionen umfasst, die den Positionen 9, 63 und 64 der SEQ ID NO: 2 entsprechen,
wobei das Polypeptid selektiv und reversibel an ein Peptid bindet, das eine Aminosäuresequenz gemäß SEQ ID NO: 3, 4 oder 5 umfasst.

2. Polypeptid nach Anspruch 1, wobei X an einer Position, die der Position 77 der SEQ ID NO: 1 oder der Position 56 der SEQ ID NO: 2 entspricht:
a) aus Alanin, Glycin oder Serin ausgewählt ist; oder
b) Alanin ist.

3. Polypeptid nach Anspruch 1 oder 2, wobei das Polypeptid eine Aminosäuresequenz mit mindestens 80 % Sequenzidentität zu einer Sequenz gemäß SEQ ID NO: 1 umfasst, und wobei die Aminosäuresequenz umfasst:
a) Histidin an einer oder beiden Positionen, die den Positionen 28 und 32 der SEQ ID NO: 1 entsprechen;
b) eines oder beide von: (i) einer Glutaminsäure an einer Position, die der Position 91 der SEQ ID NO: 1 entspricht, und (ii) einer Asparaginsäure an einer Position, die der Position 103 der SEQ ID NO: 1 entspricht; und/oder
c) eines oder mehrere von: (i) einem Threonin an einer Position, die der Position 59 der SEQ ID NO: 1 entspricht; (ii) einem Isoleucin an einer Position, die der Position 94 der SEQ ID NO: 1 entspricht, und (iii) einem Prolin an einer Position, die der Position 111 der SEQ ID NO: 1 entspricht.

4. Polypeptid nach einem der Ansprüche 1 bis 3, wobei das Polypeptid eine Aminosäuresequenz mit mindestens 80 % Sequenzidentität zu einer Sequenz gemäß SEQ ID NO: 1 umfasst und wobei die Aminosäuresequenz umfasst:
A) alles von Folgendem:
i) Histidin an Positionen, die den Positionen 28, 30, 32, 84 und 85 der SEQ D NO: 1 entsprechen;
ii) Threonin an einer Position, die der Position 59 der SEQ ID NO: 1 entspricht;
iii) Glutaminsäure an einer Position, die der Position 91 der SEQ ID NO: 1 entspricht;
iv) Isoleucin an einer Position, die der Position 94 der SEQ ID NO: 1 entspricht;
v) Asparaginsäure an einer Position, die der Position 103 der SEQ ID NO: 1 entspricht; und
vi) Prolin an einer Position, die der Position 111 der SEQ ID NO: 1 entspricht;
B) eines oder mehrere von Folgendem:
i) Lysin an einer Position, die der Position 31 der SEQ ID NO: 1 entspricht;
ii) Prolin an einer Position, die der Position 89 der SEQ ID NO: 1 entspricht;
iii) Asparaginsäure an einer Position, die der Position 97 der SEQ ID NO: 1 entspricht; und
iv) Glutaminsäure an einer Position, die der Position 108 der SEQ **ID** NO: 1 entspricht;
C) Lysin an Position 31, Prolin an Position 89, Asparaginsäure an Position 97, Glutaminsäure an Position 108 und eines oder mehrere von Folgendem:
i) Threonin an Position 2;
ii) Glycin an Position 9;
iii) Prolin an Position 13;
iv) Threonin an Position 19;
v) Arginin an Position 37;
vi) Histidin an Position 62;
vii) Glutaminsäure an Position 105; und
viii) Threonin an Position 113,
wobei sich die spezifizierten Aminosäurerückstände an Positionen befinden, die den Positionen in SEQ ID NO: 1 entsprechen; und/oder
D)
i) Threonin an Position 2;
ii) Glycin an Position 9;
iii) Prolin an Position 13;
iv) Threonin an Position 19;
v) Lysin an Position 31;
vi) Arginin an Position 37;
vii) Histidin an Position 62;
viii) Prolin an Position 89;
xiii) Asparaginsäure an Position 97;
ix) Glutaminsäure an Position 105;
xi) Glutaminsäure an Position 108; und
xii) Threonin an Position 113,
wobei sich die spezifizierten Aminosäurerückstände an Positionen befinden, die den Positionen in SEQ ID NO: 1 entsprechen.

5. Polypeptid nach einem der Ansprüche 1 bis 4, wobei das Polypeptid eine Aminosäuresequenz mit mindestens 80 % Sequenzidentität zu einer Sequenz gemäß SEQ ID NO: 1 umfasst und wobei die Aminosäuresequenz Cystein an einer Position umfasst, die der Position 49 in SEQ ID NO: 1 oder der Position 28 in SEQ ID NO: 2 entspricht.

6. Polypeptid nach einem der Ansprüche 1 bis 5, wobei das Polypeptid auf einem festen Substrat immobilisiert ist, wobei das Polypeptid vorzugsweise über eine kovalente Bindung auf dem festen Substrat immobilisiert ist, wobei das Polypeptid optional eine Aminosäuresequenz mit mindestens 80 % Sequenzidentität zu einer Sequenz gemäß SEQ ID NO: 1 umfasst und wobei die Aminosäuresequenz Cystein an Position 49 umfasst und das Polypeptid über eine kovalente Bindung zwischen dem Cystein und dem festen Substrat auf einem festen Substrat immobilisiert ist, wobei sich der spezifizierte Aminosäurerückstand an einer Position befindet, die der Position in SEQ ID NO: 1 entspricht.

7. Nukleinsäuremolekül, das eine Nukleotidsequenz umfasst, die ein Polypeptid gemäß einem der Ansprüche 1 bis 6 kodiert.

8. Vektor, der das Nukleinsäuremolekül nach Anspruch 7 umfasst.

9. Zelle, die das Nukleinsäuremolekül nach Anspruch 7 oder den Vektor nach Anspruch 8 umfasst.

10. Verfahren zum Reinigen oder Isolieren eines Moleküls oder einer Komponente, das/die ein Peptid umfasst, das eine Aminosäuresequenz mit mindestens 80 % Sequenzidentität zu einer Sequenz gemäß den SEQ ID NO: 3-5 aufweist, wobei das Peptid vorzugsweise eine Asparaginsäure an einer Position umfasst, die der Position 7 der SEQ ID NO: 3, der Position 8 der SEQ ID NO: 4 oder der Position 10 der SEQ ID NO: 5 entspricht, wobei das Verfahren Folgendes umfasst:
a) Bereitstellen eines festen Substrats, auf dem ein Polypeptid nach einem der Ansprüche 1 bis 6 immobilisiert ist;
b) Bereitstellen einer Probe, die das Molekül oder die Komponente umfasst:
c) Inkontaktbringen des festen Substrats von a) mit der Probe von b) unter Bedingungen, die es dem Peptid ermöglichen, sich selektiv an das Polypeptid zu binden, wodurch ein nicht kovalenter Komplex zwischen dem auf dem festen Substrat immobilisierten Polypeptid und dem Molekül oder der Komponente, das/die das Peptid umfasst, gebildet wird;
d) Waschen des festen Substrats mit einer Waschlösung; und
e) Trennen des Moleküls oder der Komponente, das/die das Peptid umfasst, von dem auf dem festen Substrat immobilisierten Polypeptid.

11. Verfahren nach Anspruch 10, wobei:
a) der Schritt des Inkontaktbringens des festen Substrats von a) mit der Probe von b) unter den folgenden Bedingungen erfolgt:
i) einer Temperatur von etwa 0-25 °C, vorzugsweise etwa 0-15 oder etwa 0-10 °C; und
ii) einem neutralen pH-Wert, vorzugsweise einem pH-Wert von etwa 6,5-8,5, vorzugsweise etwa 7,0-8,0 oder etwa 7,3-7,7;
b) der Schritt des Waschens des festen Substrats mit einer Waschlösung das Inkontaktbringen des festen Substrats mit einer pH-neutralen Waschlösung, optional einer gepufferten Lösung mit einem pH-Wert von etwa 6,5-8,5, vorzugsweise etwa 7,0-8,0 oder etwa 7,3-7,7, umfasst; und/oder
c) der Schritt des Trennens des Moleküls oder der Komponente, das/die das Peptid umfasst, Folgendes umfasst:
(i) Inkontaktbringen des Komplexes mit einer sauren Lösung, optional einer gepufferten Lösung mit einem pH-Wert von etwa 3,0-6,0, vorzugsweise etwa 3,5-5,5; und/oder
(ii) Erhöhen der Temperatur des Komplexes auf mindestens etwa 25 °C, vorzugsweise etwa 25-45 °C, 30-40 °C oder 35-45 °C, optional umfassend das Inkontaktbringen des Komplexes mit einer Lösung mit einer Temperatur von mindestens etwa 25 °C, vorzugsweise etwa 25-45 °C, 30-40 °C oder 35-45 °C; und/oder
(iii) Inkontaktbringen des Komplexes mit einer Lösung, die ein Peptid umfasst, das eine Aminosäuresequenz mit mindestens 80 % Sequenzidentität zu einer Aminosäuresequenz gemäß SEQ ID NO: 3-5 umfasst oder daraus besteht, vorzugsweise ein Peptid, das eine Aminosäuresequenz gemäß SEQ ID NO: 3-5 umfasst oder daraus besteht.

12. Verwendung eines Polypeptides gemäß einem der Ansprüche 1 bis 6 zum Reinigen oder Isolieren eines Moleküls oder einer Komponente, das/die ein Peptid umfasst, das eine Aminosäuresequenz mit mindestens 80 % Sequenzidentität zu einer Sequenz gemäß SEQ ID NO: 3-5 aufweist, wobei das Peptid vorzugsweise eine Asparaginsäure an einer Position umfasst, die der Position 7 der SEQ ID NO: 3, der Position 8 der SEQ ID NO: 4 oder der Position 10 der SEQ ID NO: 5 entspricht.

13. Vorrichtung, die zur Verwendung in dem Verfahren nach den Ansprüchen 10 oder 11 oder zur Verwendung nach Anspruch 12 geeignet ist, umfassend ein festes Substrat, auf dem ein Polypeptid gemäß einem der Ansprüche 1 bis 6 immobilisiert ist.

14. Kit, das zur Verwendung bei der Vorbereitung eines festen Substrats geeignet ist, auf dem ein Polypeptid gemäß einem der Ansprüche 1 bis 6 immobilisiert ist, umfassend:
a) ein Polypeptid gemäß einem der Ansprüche 1 bis 6; und
b) Mittel zum Immobilisieren des Polypeptids von a) auf einem festen Substrat, wobei das Kit optional weiter ein festes Substrat umfasst.

15. Harz und/oder Kügelchen zur Verwendung in Proteinreinigungsverfahren, bei denen ein Polypeptid nach einem der Ansprüche 1 bis 6 immobilisiert ist.

## Revendications

1. Polypeptide, comprenant :
(i) une séquence d'acides aminés telle que représentée par SEQ ID NO : 1, dans lequel X en position 77 est choisi parmi l'alanine, la glycine, la sérine, l'asparagine ou la thréonine ;
(ii) une partie de (i) comprenant une séquence d'acides aminés telle que représentée par SEQ ID NO : 2, dans lequel X en position 56 est choisi parmi l'alanine, la glycine, la sérine, l'asparagine ou la thréonine ;
(iii) une séquence d'acides aminés présentant au moins 80 % d'identité de séquence avec une séquence telle que représentée par SEQ ID NO : 1, dans lequel le polypeptide comprend de l'alanine, de la glycine, de la sérine, de l'asparagine ou de la thréonine à une position équivalente à la position 77 de SEQ ID NO : 1, et dans lequel le polypeptide comprend de l'histidine à des positions équivalentes aux positions 30, 84 et 85 de SEQ ID NO : 1 ; ou
(iv) une partie de (iii) comprenant une séquence d'acides aminés présentant au moins 80 % d'identité de séquence avec une séquence telle que représentée par SEQ ID NO : 2, dans lequel le polypeptide comprend de l'alanine, de la glycine, de la sérine, de l'asparagine ou de la thréonine à une position équivalente à la position 56 de SEQ ID NO : 2, et dans lequel le polypeptide comprend de l'histidine à des positions équivalentes aux positions 9, 63 et 64 de SEQ ID NO : 2,
dans lequel le polypeptide se lie de manière sélective et réversible à un peptide comprenant une séquence d'acides aminés telle que représentée par SEQ ID NO : 3, 4 ou 5.

2. Polypeptide selon la revendication 1, dans lequel X à une position équivalente à la position 77 de SEQ ID NO : 1 ou à la position 56 de SEQ ID NO : 2 :
a) est choisi parmi l'alanine, la glycine ou la sérine ; ou
b) est une alanine.

3. Polypeptide selon la revendication 1 ou 2, dans lequel le polypeptide comprend une séquence d'acides aminés présentant au moins 80 % d'identité de séquence avec une séquence telle que représentée par SEQ ID NO : 1, et dans lequel la séquence d'acides aminés comprend :
a) une histidine à une position ou à deux positions équivalentes aux positions 28 et 32 de SEQ ID NO : 1 ;
b) un ou les deux parmi : (i) un acide glutamique à une position équivalente à la position 91 de SEQ ID NO : 1 ; et (ii) un acide aspartique à une position équivalente à la position 103 de SEQ ID NO : 1 ; et/ou
c) une ou plusieurs parmi : (i) une thréonine à une position équivalente à la position 59 de SEQ ID NO : 1 ; (ii) une isoleucine à une position équivalente à la position 94 de SEQ ID NO : 1 ; et (iii) une proline à une position équivalente à la position 111 de SEQ ID NO : 1.

4. Polypeptide selon l'une quelconque des revendications 1 à 3, dans lequel le polypeptide comprend une séquence d'acides aminés présentant au moins 80 % d'identité de séquence avec une séquence telle que représentée par SEQ ID NO : 1 et dans lequel la séquence d'acides aminés comprend :
A) l'ensemble des suivants :
i) une histidine à des positions équivalentes aux positions 28, 30, 32, 84 et 85 de SEQ ID NO : 1 ;
ii) une thréonine à une position équivalente à la position 59 de SEQ ID NO : 1 ;
iii) un acide glutamique à une position équivalente à la position 91 de SEQ ID NO : 1 ;
iv) une isoleucine à une position équivalente à la position 94 de SEQ ID NO : 1 ;
v) un acide aspartique à une position équivalente à la position 103 de SEQ ID NO : 1 ; et
vi) une proline à une position équivalente à la position 111 de SEQ ID NO : 1 ;
B) un ou plusieurs des suivants :
i) une lysine à une position équivalente à la position 31 de SEQ ID NO : 1 ;
ii) une proline à une position équivalente à la position 89 de SEQ ID NO : 1 ;
iii) un acide aspartique à une position équivalente à la position 97 de SEQ ID NO : 1 ; et
iv) un acide glutamique à une position équivalente à la position 108 de SEQ ID NO : 1 ;
C) une lysine en position 31, une proline en position 89, un acide aspartique en position 97, un acide glutamique en position 108 et un ou plusieurs des suivants :
i) une thréonine en position 2 ;
ii) une glycine en position 9 ;
iii) une proline en position 13 ;
iv) une thréonine en position 19 ;
v) une arginine en position 37 ;
vi) une histidine en position 62 ;
vii) un acide glutamique en position 105 ; et
viii) une thréonine en position 113,
dans lequel les résidus d'acide aminé spécifiés sont à des positions équivalentes aux positions dans SEQ ID NO : 1 ; et/ou
D)
i) une thréonine en position 2 ;
ii) une glycine en position 9 ;
iii) une proline en position 13 ;
iv) une thréonine en position 19 ;
v) une lysine en position 31 ;
vi) une arginine en position 37 ;
vii) une histidine en position 62 ;
viii) une proline en position 89 ;
ix) un acide aspartique en position 97 ;
x) un acide glutamique en position 105 ;
xi) un acide glutamique en position 108 ; et
xii) une thréonine en position 113,
dans lequel les résidus d'acide aminé spécifiés sont à des positions équivalentes aux positions dans SEQ ID NO : 1.

5. Polypeptide selon l'une quelconque des revendications 1 à 4, dans lequel le polypeptide comprend une séquence d'acides aminés présentant au moins 80 % d'identité de séquence avec une séquence telle que représentée par SEQ **ID** NO : 1 et dans lequel la séquence d'acides aminés comprend une cystéine à une position équivalente à la position 49 dans la SEQ ID NO : 1 ou à la position 28 dans la SEQ ID NO : 2.

6. Polypeptide selon l'une quelconque des revendications 1 à 5, dans lequel le polypeptide est immobilisé sur un substrat solide, de préférence dans lequel le polypeptide est immobilisé sur le substrat solide par l'intermédiaire d'une liaison covalente, facultativement dans lequel le polypeptide comprend une séquence d'acides aminés présentant au moins 80 % d'identité de séquence avec une séquence telle que représentée par SEQ ID NO : 1 et dans lequel la séquence d'acides aminés comprend une cystéine en position 49 et le polypeptide est immobilisé sur un substrat solide par l'intermédiaire d'une liaison covalente entre ladite cystéine et le substrat solide, dans lequel le résidu d'acide aminé spécifié est à une position équivalente à la position dans la SEQ ID NO : 1.

7. Molécule d'acide nucléique comprenant une séquence nucléotidique qui code pour un polypeptide tel que défini dans l'une quelconque des revendications 1 à 6.

8. Vecteur comprenant la molécule d'acide nucléique selon la revendication 7.

9. Cellule comprenant la molécule d'acide nucléique selon la revendication 7 ou le vecteur selon la revendication 8.

10. Procédé de purification ou d'isolation d'une molécule ou d'un composant comprenant un peptide présentant une séquence d'acides aminés présentant au moins 80 % d'identité de séquence avec une séquence telle que représentée par l'une des SEQ ID NO : 3 à 5, de préférence dans lequel ledit peptide comprend un acide aspartique à une position équivalente à la position 7 de SEQ ID NO : 3, à la position 8 de SEQ ID NO : 4 ou à la position 10 de SEQ ID NO : 5, ledit procédé comprenant :
a) la fourniture d'un substrat solide sur lequel est immobilisé un polypeptide selon l'une quelconque des revendications 1 à 6 ;
b) la fourniture d'un échantillon comprenant ladite molécule ou ledit composant ;
c) la mise en contact du substrat solide de a) avec l'échantillon de b) dans des conditions qui permettent audit peptide de se lier sélectivement audit polypeptide, pour ainsi former un complexe non covalent entre ledit polypeptide immobilisé sur le substrat solide et la molécule ou le composant comprenant ledit peptide ;
d) le lavage du substrat solide avec une solution de lavage ; et
e) la séparation de la molécule ou du composant comprenant le peptide à partir du polypeptide immobilisé sur le substrat solide.

11. Procédé selon la revendication 10, dans lequel :
a) l'étape de mise en contact du substrat solide de a) avec l'échantillon de b) a lieu dans les conditions suivantes :
i) une température d'environ 0 à 25 °C, de préférence d'environ 0 à 15 ou d'environ 0 à 10 °C ; et
ii) un pH neutre, de préférence un pH d'environ 6,5 à 8,5, de préférence d'environ 7,0 à 8,0 ou d'environ 7,3 à 7,7 ;
b) l'étape de lavage du substrat solide avec une solution de lavage comprend la mise en contact dudit substrat solide avec une solution de lavage à pH neutre, facultativement une solution tamponnée ayant un pH d'environ 6,5 à 8,5, de préférence d'environ 7,0 à 8,0 ou d'environ 7,3 à 7,7 ; et/ou
c) l'étape de séparation de la molécule ou du composant comprenant le peptide comprend :
(i) la mise en contact dudit complexe avec une solution acide, facultativement une solution tamponnée ayant un pH d'environ 3,0 à 6,0, de préférence d'environ 3,5 à 5,5 ; et/ou
(ii) l'augmentation de la température dudit complexe jusqu'à au moins environ 25 °C, de préférence environ 25 à 45 °C, 30 à 40 °C ou 35 à 45 °C, facultativement comprenant la mise en contact dudit complexe avec une solution présentant une température d'au moins environ 25 °C, de préférence d'environ 25 à 45 °C, 30 à 40 °C ou 35 à 45 °C ; et/ou
(iii) la mise en contact dudit complexe avec une solution comprenant un peptide comprenant ou consistant en une séquence d'acides aminés présentant au moins 80 % d'identité de séquence avec une séquence d'acides aminés telle que représentée par l'une des SEQ ID NO : 3 à 5, de préférence un peptide comprenant ou consistant en une séquence d'acides aminés telle que représentée par l'une des SEQ ID NO : 3 à 5.

12. Utilisation d'un polypeptide tel que défini dans l'une quelconque des revendications 1 à 6 pour purifier ou isoler une molécule ou un composant comprenant un peptide présentant une séquence d'acides aminés présentant au moins 80 % d'identité de séquence avec une séquence telle que représentée par l'une des SEQ ID NO : 3 à 5, de préférence dans laquelle ledit peptide comprend un acide aspartique à une position équivalente à la position 7 de SEQ ID NO : 3, à la position 8 de
SEQ ID NO : 4 ou à la position 10 de SEQ ID NO : 5.

13. Appareil approprié pour une utilisation dans le procédé selon les revendications 10 ou 11 ou pour une utilisation selon la revendication 12 comprenant un substrat solide sur lequel est immobilisé un polypeptide tel que défini dans l'une quelconque des revendications 1 à 6.

14. Kit approprié pour une utilisation dans la préparation d'un substrat solide sur lequel est immobilisé un polypeptide tel que défini dans l'une quelconque des revendications 1 à 6, comprenant :
a) un polypeptide tel que défini dans l'une quelconque des revendications 1 à 6 ; et
b) des moyens pour immobiliser le polypeptide d'a) sur un substrat solide, facultativement dans lequel le kit comprend en outre un substrat solide.

15. Résine et/ou billes, pour une utilisation dans des procédés de purification de protéine, sur lesquelles est immobilisé un polypeptide tel que défini dans l'une quelconque des revendications 1 à 6.
